# EUROPEAN PATENT SPECIFICATION

(11) **EP 4 281 778 B1**
(45) Date of publication and mention of the grant of the patent: **17.09.2025**
(21) Application number: 22703691.0
(22) Date of filing: 24.01.2022
(51) Int. Cl.: G01N 33/574

(54) **USE OF A FUC-ALPHA(1-2)-GAL GLYCAN STRUCTURE AS A BIOMARKER FOR BLADDER CANCER**
VERWENDUNG EINER FUC-ALPHA(1-2)-GAL GLYKAN STRUKTUR ALS BIOMARKER FÜR BLASENKREBS
UTILISATION D'UNE STRUCTURE GLYCANE FUC-ALPHA(1-2)-GAL COMME BIOMARQUEUR DU CANCER DE LA VESSIE

(30) Priority: 25.01.2021 FI 20215078
(43) Date of publication of application: 29.11.2023
(73) Proprietor: Uniogen Oy, 20520 Turku (FI)
(72) Inventor: ISLAM, Md Khirul, 20520 Turku (FI); SYED, Parvez, 20520 Turku (FI); LEIVO, Janne, 20520 Turku (FI); GIDWANI, Kamlesh, 20520 Turku (FI)
(74) Representative: Primrose Oy
(86) International application number: PCT/FI2022/050043
(87) International publication number: WO 2022/157422

(56) References cited:
- LU YI-CHIEN ET AL: "Calreticulin activates [beta]1 integrin via fucosylation by fucosyltransferase 1 in J82 human bladder cancer cells.", THE BIOCHEMICAL JOURNAL 15 MAY 2014, vol. 460, no. 1, 15 May 2014 (2014-05-15), pages 69 - 78, XP009534740, ISSN: 1470-8728
- LI CHONG ET AL: "BCMab1, A Monoclonal Antibody against Aberrantly Glycosylated Integrin [alpha]3[beta]1, Has Potent Antitumor Activity of Bladder Cancer In Vivo", CLINICAL CANCER RESEARCH, vol. 20, no. 15, 7 July 2014 (2014-07-07), US, pages 4001 - 4013, XP055908742, ISSN: 1078-0432, Retrieved from the Internet <URL:https://com-mendeley-prod-publicsharing-pdfstore.s3.eu-west-1.amazonaws.com/1e17-CC-BY-2/10.1158/1078-0432.ccr-13-3397.pdf?X-Amz-Security-Token=IQoJb3JpZ2luX2VjEN7//////////wEaCWV1LXdlc3QtMSJHMEUCIGU3y0iP9ci0hKeew6TB3zBBvHrcmPHURSkR36c06UrnAiEA7A0GY4KaKxqgcDT1s2S3wk13bv4yeSqiLW9Pwjf31GwqgwQIdxADGgwx> DOI: 10.1158/1078-0432.CCR-13-3397
- OKAMURA TAKEHIKO ET AL: "Lectin immunohistochemical evaluation of human bladder carcinomas. A comparison of Carnoy's and formalin fixation.", HINGYOKIKA KIYO - ACTA UROLOGICA JAPONICA., vol. 39, no. 10, 1 October 1993 (1993-10-01), JP, pages 899 - 905, XP055908777, ISSN: 0018-1994, Retrieved from the Internet <URL:https://repository.kulib.kyoto-u.ac.jp/dspace/bitstream/2433/117959/1/39_899.pdf>
- JUHL BIRGITTE RAVN ET AL: "A, B, H antigen expression in transitional cell carcinomas of the urinary bladder", CANCER, vol. 57, no. 9, 1 May 1986 (1986-05-01), US, pages 1768 - 1775, XP055908820, ISSN: 0008-543X, DOI: 10.1002/1097-0142(19860501)57:9<1768::AID-CNCR2820570910>3.0.CO;2-E
- ISLAM MD KHIRUL ET AL: "Detection of bladder cancer with aberrantly fucosylated ITGA3", ANALYTICAL BIOCHEMISTRY, ACADEMIC PRESS, AMSTERDAM, NL, vol. 628, 5 June 2021 (2021-06-05), XP086707536, ISSN: 0003-2697, [retrieved on 20210605], DOI: 10.1016/J.AB.2021.114283

## Description

### FIELD OF THE INVENTION

The present disclosure relates to non-invasive detection of bladder cancer on the basis of altered glycosylation patterns of cancer-associated biomarkers, especially integrins.

### BACKGROUND OF THE INVENTION

Overexpression of proteins is a typical feature of cancer cells. As such, tumor markers, each indicative of a particular disease process, may be utilized in oncology to help detect the presence of cancer. Examples of such tumor markers include various integrins and mucins.

In addition to overexpression, aberrant glycosylation of proteins is a frequently observed phenomenon in cancers. This is not surprising since glycosylation is involved in several physiological processes regulating the development and progression of cancer. For example, glycans play a role in cell signaling, cell-matrix interactions, tumor cell dissociation and invasion, metastasis formation, angiogenesis and immune modulation. The most common cancer-associated changes in protein glycosylation are increased sialylation, increased branched-glycan structures and overexpression of core fucosylation. For example, overexpression of N-acetylglucosamine (GlcNAc) and oversialylation and/or overexpression of sialylated Lewis antigens such as sialyl-Lewis^{a} (sLe^{a}) and sialyl-Lewis^{x} (sLe^{x}) have been reported. As a further example, certain glycovariants of mucin 1 (MUC1) have been reported to be present in elevated levels in majority of breast cancers. Moreover, modifications of integrins with branched N-glycans, truncated O-glycans and/or sialylated structures have been reported to modulate tumor cell-matrix interactions, promoting tumor cell migration.

The present disclosure relates to bladder cancer (BlCa), which is the second most common urological malignancy in men after prostate cancer. As of 2015, BlCa affects almost 3.4 million people, with 430,000 new cases found annually. BlCa is the seventh most commonly diagnosed cancer worldwide for men and the eleventh most commonly diagnosed cancer when both genders are considered. Although the occurrence of BlCa is not the highest among urogenital cancers, the costs involved in management of the disease makes it the most expensive cancer. One of the main reasons for such high costs is that approximately 70% of patients in all stages of the diseases recur within 5 years. Symptoms for bladder cancer include hematuria and irritative voiding. Currently, the diagnosis and surveillance of bladder cancer is dependent on voided urine cytology and direct cystoscopic examination of the bladder. However, cystoscopy is an invasive, unpleasant and costly procedure and can miss small papillary tumors or carcinoma *in situ* lesions. Urinary cytology has a low sensitivity for low-grade tumors and negative cytology does not exclude the presence of a tumor. Numerous biomarkers/kits for the detection of BlCa have been developed. These include assays based on the detection of bladder tumor antigen (BTA), nuclear matrix protein 22 (NMP22) and the ImmunoCyt+ test which is based on detection of mucins and carcinoembryonic antigen (CEA). However, all of these tests have limitations in either sensitivity or specificity and none of them have been accepted for the diagnosis or follow-up of BlCa in routine practice or clinical guidelines. Since BlCa is a genitourinary tract cancer, soluble and membrane bound glycoproteins which are released into the urine serve as an attractive target for developing non-invasive diagnostic tools.

Yi-Chien Lu et al. (Biochem. J., 2014, 460(1): 69-78) discloses studies of the glycosylation of β1 integrin by western blot analysis with an anti-β1 integrin antibody and *Ulex europaeus agglutinin-1* (UEA-1), and concludes that α1,2-fucosylation of β1 integrin affects the cell adhesion ability of a human J82 bladder cancer cell line. The conclusion is based on a treatment of the β1 integrin with a fucosidase to remove the α1,2-linked fucose residues, which treatment results in a reduction of the adhesion of the J82 bladder cancer cells.

Li Chong et al. (Clin. Cancer Res., 2014, 20(15): 4001-4013) suggests a monoclonal antibody against aberrantly glycosylated integrin α3β1 as a biomarker for bladder cancer. The carbohydrate composition of the oligosaccharides in the integrin α3β1 epitope that reacts positively with the antibody is [3OSO3]Galβ1-4(Fucα1-3)[6OSO3]GlcNAc.

Okamura Takehiko et al. (Acta Urol. Jpn., 1993, 39(10): 899-905) discloses lectin immunohistochemical analysis of 51 human bladder carcinomas. Tissue samples of transitional cell carcinoma prepared with Carnoy's fixation were moderately positive for UEA-1. Also Juhl Birgitte Ravn et al. (Cancer, 1986, 57(9): 1768-1775) discloses UEA-1 for immunohistochemical analysis of bladder cancer.

There is still an urgent need for the development of a non-invasive, sensitive and specific BlCa biomarker various diagnostic and prognostic purposes.

### BRIEF DESCRIPTION OF THE INVENTION

An aspect of the present invention provides use of a FUCα(1-2)Gal glycan structure as a biomarker for bladder cancer, wherein said FUCα(1-2)Gal glycan structure is comprised on an integrin-presenting vesicle or an integrin antigen, wherein the integrin is selected from the group consisting of integrin subunit alpha 3 (ITGA3), integrin subunit alpha 1 (ITGA1), integrin subunit alpha 5 (ITGA5), integrin subunit alpha 11 (ITGA11), and integrin subunit alpha V (ITGAV).

Another aspect of the present invention provides a method of determining bladder cancer disease state in a subject, the method comprising:
a) assaying a sample of a bodily fluid obtained from said subject for the level of integrin-presenting vesicles comprising a FUCα(1-2)Gal glycan structure, or for the level of integrin antigen comprising a FUCα(1-2)Gal glycan structure, wherein the integrin is selected from the group consisting of integrin subunit alpha 3 (ITGA3), integrin subunit alpha 1 (ITGA1), integrin subunit alpha 5 (ITGA5), integrin subunit alpha 11 (ITGA11), and integrin subunit alpha V (ITGAV),
b) comparing the assayed level obtained in step a) with that of a control sample or a predetermined threshold value, and
c) determining the cancer disease state on the basis of said comparison.

According to an embodiment of the above-mentioned aspects, increased level of said integrin antigen or said integrin-presenting vesicle comprising the FUCα(1-2)Gal glycan structure as compared with that of the control sample or predetermined threshold value indicates that said subject has or is at risk of having bladder cancer.

According to an embodiment of the method, said assaying is carried out by using a binder molecule specific for a FUCα(1-2)Gal glycan structure or by using mass spectrometry, nuclear magnetic resonance (NMR) spectroscopy, electrophoresis, chromatography or a combination thereof. According to an embodiment, the binder molecule is *Ulex europaeus* agglutinin-I (UEA-I) or an antibody specific for the FUCα(1-2)Gal glycan structure.

According to a further embodiment, the method further comprises assaying said sample for mucin 1 (MUC1) capable of binding to wheat germ agglutinin (WGA) (MUC1^{WGA}), MUC1 capable of binding to anti-Tn antigen antibody (Tn-AB) (MUC1^{Tn-AB}), and/or MUC1 capable of binding to *Helix pomatia* agglutinin (HPA) (MUC1^{HPA}).

A further aspect of the present invention provides a kit for use in determining cancer disease state in a subject. The kit comprises an integrin-binding agent and a binder molecule specific for a FUCα(1-2)Gal glycan structure. Either said integrin-binding agent or said binder molecule comprises a detectable label. Furtthermore, the integrin is selected from the group consisting of integrin subunit alpha 3 (ITGA3), integrin subunit alpha 1 (ITGA1), integrin subunit alpha 5 (ITGA5), integrin subunit alpha 11 (ITGA11), and integrin subunit alpha V (ITGAV).

A still further aspect of the present invention provides use of a kit for determining bladder cancer disease state in a subject. The kit comprises: an integrin-binding agent and a binder molecule specific for a FUCα(1-2)Gal glycan structure, wherein either said antigen-binding agent or said binder molecule comprises a detectable label. Furthermore, the integrin is selected from the group consisting of integrin subunit alpha 3 (ITGA3), integrin subunit alpha 1 (ITGA1), integrin subunit alpha 5 (ITGA5), integrin subunit alpha 11 (ITGA11), and integrin subunit alpha V (ITGAV).

An even further aspect of the present invention provides use of a binder molecule specific for a FUCα(1-2)Gal glycan structure for determining the presence or absence of a bladder cancer-associated integrin glycoform in a sample of a bodily fluid.

In an embodiment of the above-mentioned aspects, the binder molecule is UEA-I or an antibody specific for the FUCα(1-2)Gal glycan structure.

### BRIEF DESCRIPTION OF THE DRAWINGS

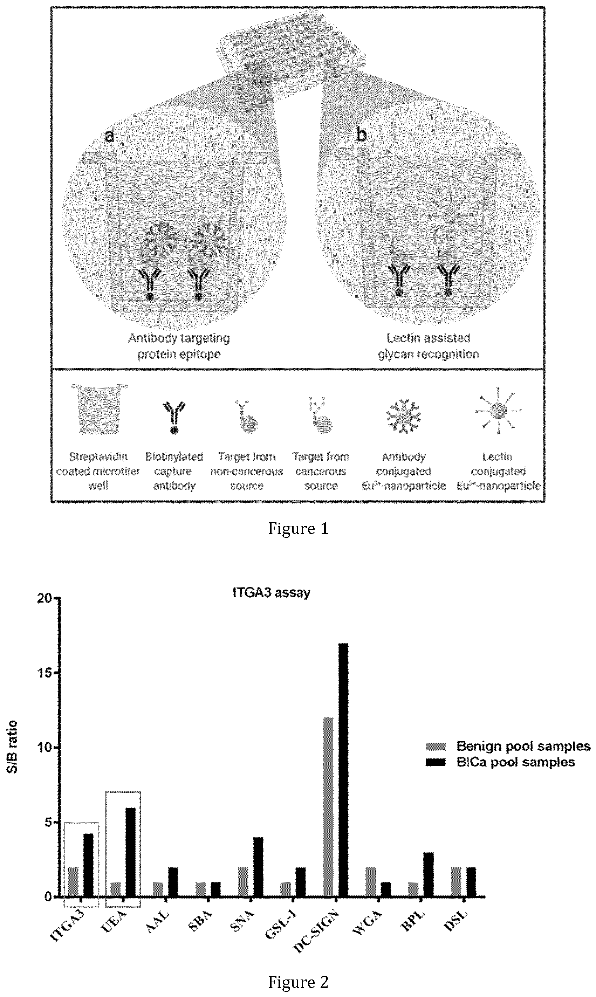

In the following the invention will be described in greater detail by means of preferred embodiments with reference to the accompanying drawings, in which
Figure 1 illustrates a schematic representation of assaying glycoforms from cancerous and non-cancerous sources. Biotinylated antibodies immobilized on the surface of streptavidin coated microtiter wells capture (glyco)proteins from samples. The glycans on captured proteins are detected with Eu³⁺-doped polystyrene nanoparticles conjugated either with anti-protein antibodies (Part a) or lectin(s) (Part b).
Figure 2 shows analysis of pooled urine samples for ITGA3 glycoforms derived from bladder cancer patients and benign (BPH) controls. The binding of different lectins to ITGA3 derived from BPH and BlCa samples is represented as signal to background (S/B) ratios.

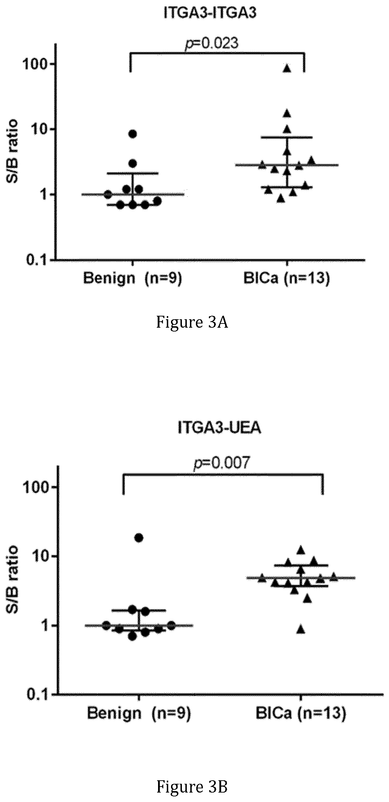

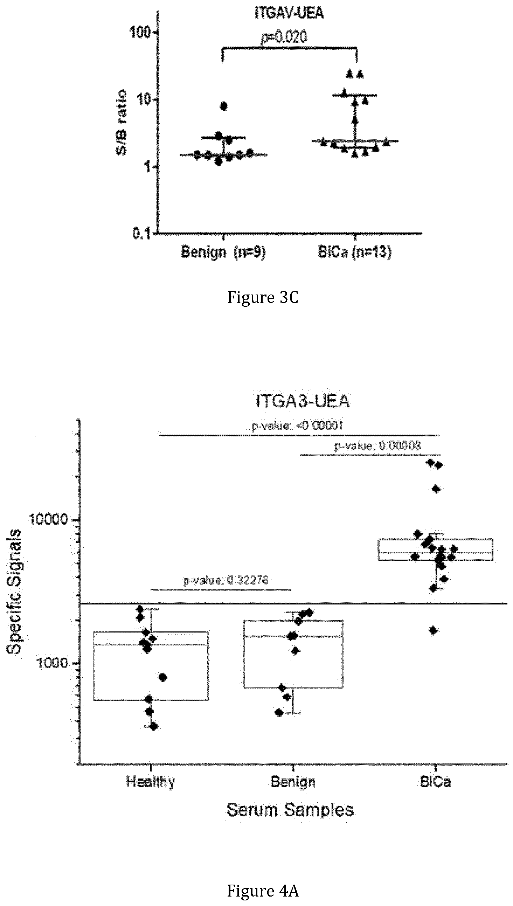

Figure 3 illustrates analysis of urine samples for ITGA3 derived from bladder cancer patients (n=13) and benign controls (n=9) with a conventional ITGA3-ITGA3 immunoassay (Figure 3A) or ITGA3^{UEA-I} assay (Figure 3B). Analysis of the same samples for UEA-I binding glycoforms of ITGAV (i.e. ITGAV^{UEA-I}) is illustrated in Figure 3C.

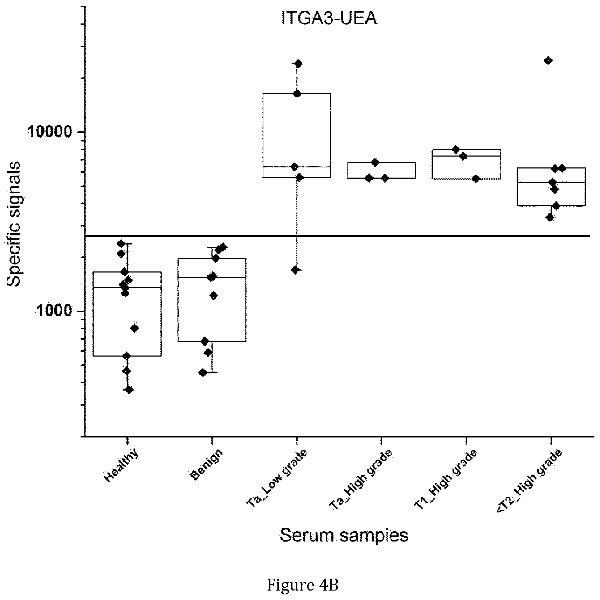

Figure 4A illustrates analysis with ITGA3^{UEA-I} assay of serum samples from apparently healthy patients (n=11), benign urological disease patients (n=9) and BlCa patients (n=18). In Figure 4B, of the total of 18 BlCa samples, 5, 3, 3 and 7 samples were classified as Ta low grade, Ta high grade, T1 high grade and <T2 high grade, respectively. Tumors classified as Ta and T1 were considered non-muscle invasive bladder cancer (NMIBC) and tumors classified as <T2 were considered muscle invasive bladder cancer (MIBC). The ITGA3^{UEA-I} assay successfully discriminated BlCa from healthy volunteers (p-value: <0.00001) (Figure 4A) and was also able to detect both NMBIC and MIBC samples (Figure 4B).

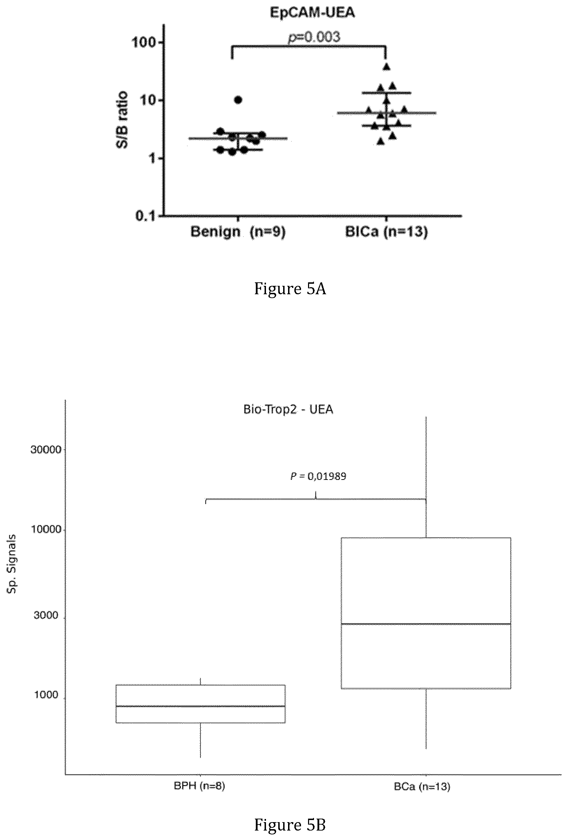

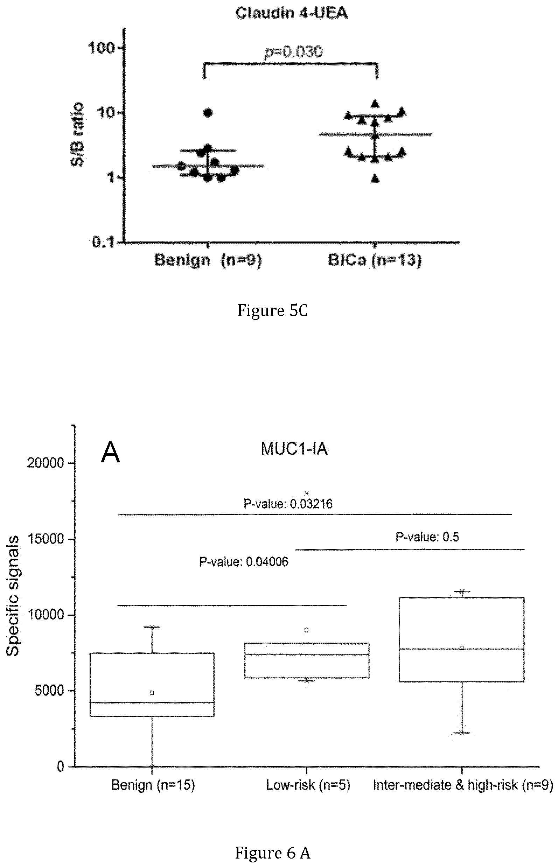

Figure 5 illustrates analysis of urine samples for UEA-I binding glycoforms of EpCAM (Figure 5A), anti-Trop2 (Figure 5B) or anti-claudin-4 (Figure 5C) derived from bladder cancer patients (n=13) and benign controls (n=9).

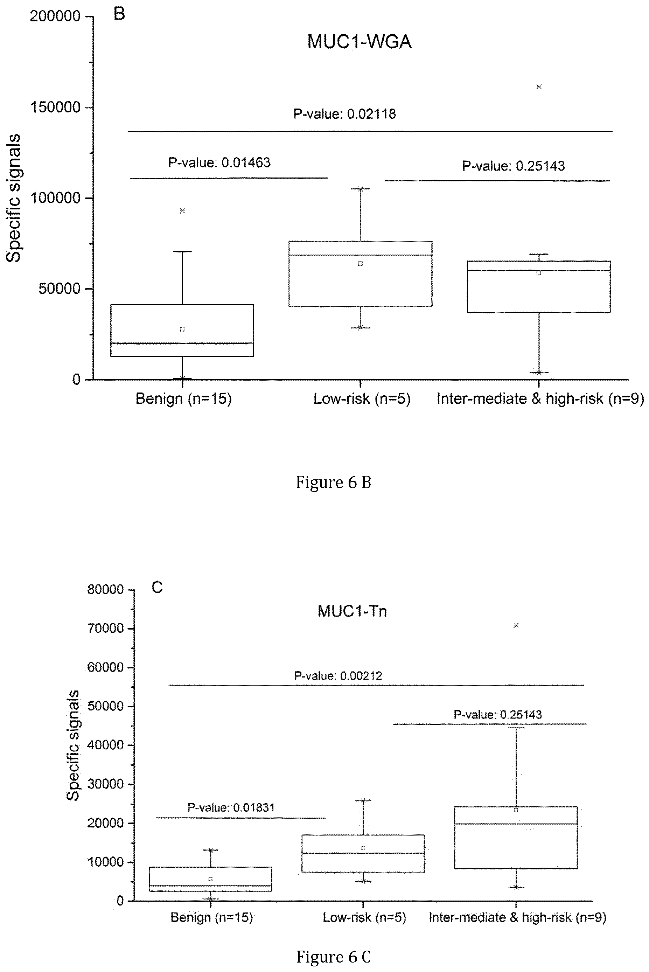

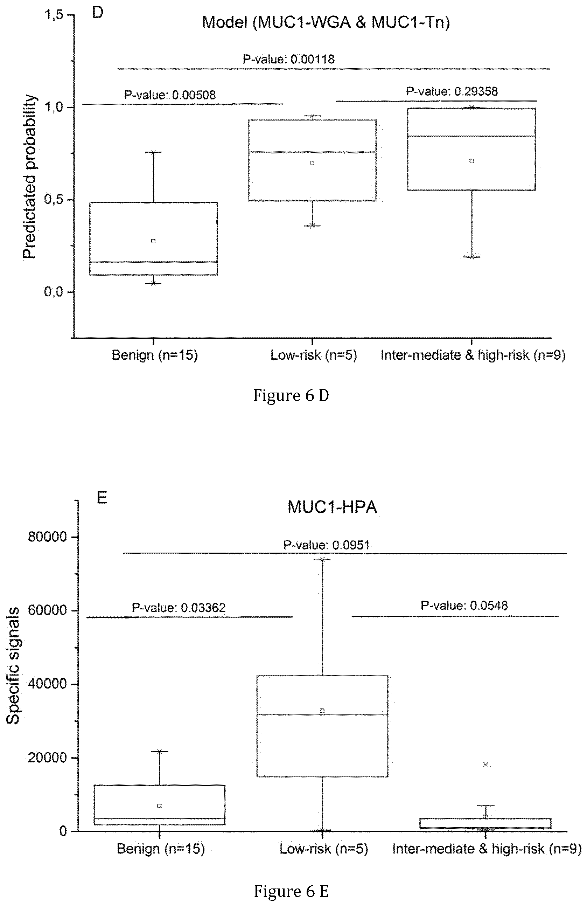

Figure 6 illustrates assays of urine samples from benign urological disease patients and patients with low-risk or intermediate & high-risk bladder cancer. Figure 6A represents a conventional MUC1 immunoassay using antibodies conjugated with Eu³⁺ chelates as tracers. Figure 6B shows a MUC1-WGA lectin assay (MUC1^{WGA}). Figure 6C represents a MUC1 anti-Tn antibody assay (MUC1^{Tn-AB}) using an anti-Tn antibody conjugated to Eu³⁺-doped nanoparticle as tracer. Figure 6D represents a logistic regression model combining data of assays of Figures 6B and 6C. Figure 6E represents a MUC1-HPA lectin assay (MUC1^{HPA}).

### DEFINITIONS

Unless otherwise defined, the terms and expressions used in this specification and claims have the meanings generally applicable in the field of cancer diagnostics. Some of terms and expression used herein are have the meanings as defined below:
As used herein, the meaning of a singular noun includes that of a plural noun and thus a singular term, unless otherwise specified, may also carry the meaning of its plural form. In other words, the term "a" or "an" may mean one or more.

As used herein, the term "or" in the claims is used to mean "and/or" unless explicitly indicated to refer to alternatives only or when the alternatives are mutually exclusive, although the disclosure supports a definition that refers to only alternatives and "and/or."

As used herein, the terms "biological sample" and "sample" are interchangeable and refer to a sample of a bodily fluid, such as ascites fluid, urine, blood, plasma, serum, and peritoneal cavity fluid, obtained from a subject. Generally, obtaining the sample to be analysed from a subject is not part of the present method for determining a subject's cancer disease state. A urine, blood, serum or plasma sample is the most preferred sample type to be used in the present method and all its embodiments. In some embodiments, the sample is an EDTA plasma sample.

In embodiments which concern assessment of the level of more than one biomarker, same or different samples obtained from a subject whose cancer disease state is to be determined may be used for each assessment. Said different samples may be of the same or different type.

As used herein, the terms "biomarker" and "marker" are interchangeable, and refer broadly to a molecule which is differentially present in a sample taken from a subject with cancer as compared to a comparable sample take from a control subject, such as an apparently healthy subject. More specifically, the terms refer to certain glycovariants of antigens indicative of a given cancer.

As used herein, the term "antigen" refers broadly to any herein-disclosed protein antigen indicative of cancer. In some embodiments, the antigen is integrin (ITG) or any subunit thereof, epithelium cell adhesion molecule (EpCAM), claudin-4, tumor-associated calcium signal transducer 2 (Trop2) or mucin 1 (MUC1).

As used herein, the term "antigen entity" refers broadly to any herein-disclosed antigen in soluble form and to an extracellular vesicle which presents said antigen on its surface. Thus, the term "integrin entity", for example, encompasses soluble integrins as well as integrin-presenting extracellular vesicles.

As used herein, the terms "glycoform" and "glycovariant" are interchangeable and refer to a particular form of a glycosylated biomarker. That is, when the same protein backbone that is part of a biomarker has the potential to be linked to different glycans or sets of glycans, then each different version of the biomarker is referred to as a "glycoform".

As used herein, the term "binder molecule" refers to broadly to any molecule that is able bind a biomarker or a glycan part thereof. More specifically, the term "glycan binder", and the like, refer to any glycan-specific binder molecule. Non-limiting examples of binder molecules include lectins, antibodies, antibody mimetics, and oligonucleotide and peptide aptamers. Glycan binders for use in the present invention are specific for a fucosylated glycan structure, namely FUCα(1-n) glycan structure, wherein n is 2, more specifically FUCα(1-2)Gal. As used herein, "Gal" refers to galactose. In some embodiments FUCα(1-2)Gal refers to terminal fucosylation.

As used herein, the term "antibody" refers to an immunoglobulin structure comprising two heavy (H) chains and two light (L) chains interconnected by disulphide bonds. Antibodies can exist as intact immunoglobulins or as any of a number of well-characterized antigen-binding fragments or single chain variants thereof, all of which are herein encompassed by the term "antibody". Non-limiting examples of said antigen-binding fragments include Fab fragments, Fab' fragments, F(ab')2 fragments, and Fv fragments. Said fragments and variants may be produced by recombinant DNA techniques, or by enzymatic or chemical separation of intact immunoglobulins as is well known in the art.

As used herein, the term "subject" refers to an animal, preferably to a mammal, more preferably to a human, and in some embodiments most preferably to a female, while in some other embodiments most preferably to a male. Depending on an embodiment in question, said subject may suffer from cancer with or without diagnosis, be suspected to suffer from cancer, be at risk of cancer, or may have already been treated for cancer. Herein, the terms "human subject", "patient" and "individual" are interchangeable.

As used herein, the term "apparently healthy" refers to an individual or a pool of individuals who show no signs or symptoms of cancer or a benign condition and thus are believed not to be affected by cancer or a benign condition or who are predicted not to develop cancer or a benign condition. Herein, the terms "apparently healthy" and "healthy" may in some instances be used interchangeably.

As used herein, terms such as "benign condition" and the like refer to non-cancerous conditions. Similarly, as used herein, the terms "benign urological condition", "benign urological disease" and the like refer to non-cancerous urological conditions including but not limited to benign prostatic hyperplasia. Similarly, as used herein, terms such as "benign control" and "benign cohort" and the like refer to subjects or patients having a benign condition and/or to samples from subjects or patients having a benign condition. Those skilled in the art readily understand which benign conditions are appropriate to consider which respect to a given cancer.

As used herein, the term "benign prostatic hyperplasia" (BPH), also known as benign prostatic hypertrophy, refers to a common noncancerous enlargement of the prostate gland in aging men. Generally, BPH is considered neither as a premalignant lesion nor a precursor of carcinoma.

As used herein, the term "indicative of cancer", when applied to a biomarker, refers to a level which, using routine statistical methods setting confidence levels at a minimum of 95%, is diagnostic of said cancer or a stage of said cancer such that the detected level is found significantly more often in subjects with said cancer or a stage of said cancer than in subjects without said cancer or another stage of said cancer. Preferably, the level which is indicative of cancer is found in at least 80% of subjects who have the cancer and is found in less than 10% of subjects who do not have the cancer. More preferably, the level which is indicative of said cancer is found in at least 90%, at least 95%, at least 98%, or more in subjects who have the cancer and is found in less than 10%, less than 8%, less than 5%, less than 2.5%, or less than 1% of subjects who do not have the cancer.

As used herein, the term "level" is interchangeable with the terms "amount" and "concentration", unless otherwise indicated.

To determine whether a detected level of a biomarker is indicative of the presence or risk of the presence of a cancer associated with said biomarker, its level in a relevant control has to be determined. Once the control levels are known, the determined marker levels can be compared therewith and the significance of the difference can be assessed using standard statistical methods. In some embodiments, a statistically significant difference between the determined biomarker level and the control level is indicative of the cancer in question. In some further embodiments, before to be compared with the control, the biomarker levels are normalized using standard methods.

Comparison of the assayed level of a biomarker in a sample to be analysed with that of a relevant control or a predetermined threshold value may in some embodiments be performed by a processor of a computing device.

Regardless of whether or not the processor of the computing device is used for said comparison, the level of the assayed level of a biomarker is, at least in some embodiments, determined as "increased" or "higher" if the level of the biomarker in the sample is, for instance, at least about 1.5 times, at least about 1.75 times, at least about 2 times, at least about 3 times, at least about 4 times, at least about 5 times, at least about 6 times, at least about 8 times, at least about 9 times, at least about 10 times, at least about 20 times or at least about 30 times the predetermined threshold level or the level of the biomarker in a control sample. In some embodiments, the difference between the level of the biomarker in the sample to be analyzed and the predetermined threshold level or the level of the biomarker in a control sample has to be statistically significant in order to provide a proper diagnostic, prognostic or predictive result.

Concentration of a biomarker in a sample obtained from a subject whose cancer disease state is to be determined or who is to be screened, diagnosed, prognosed, or monitored for cancer is considered "non-increased" or "normal" if the detected concentration thereof is lower, essentially the same or essentially non-altered as compared with that of a relevant control sample or a predetermined threshold value.

As used herein, the term "control" may refer to a control sample obtained from an apparently healthy individual or pool of apparently healthy individuals, or it may refer to a control sample obtained from an individual or a pool of individuals with a benign condition such as a benign urological condition such as benign prostatic hyperplasia, or it may refer to a predetermined threshold value, i.e. a cut-off value, which is indicative of the presence or absence of bladder cancer. Statistical methods for determining appropriate threshold values will be readily apparent to those of ordinary skill in the art. The threshold values may have been determined, if necessary, from samples of subjects of the same age, demographic features, and/or disease status, etc. The threshold value may originate from a single individual not affected by the cancer in question or be a value pooled from more than one such individual.

In some embodiments, the term "control sample" refers to a sample obtained from the same subject whose cancer disease state is to be determined but obtained at a time point different from the time point of the disease state determination. Non-limiting examples of such different time points include one or more time points before diagnosis of the disease, one or more time points after diagnosis of the disease, one or more time points before treatment of the disease, one or more time points during treatment of the disease, and one or more time points after treatment of the disease. Typically, such control samples obtained from the same subject are used when the purpose of cancer disease state determination is to monitor said disease, especially to monitor the onset of the disease, or risk development of the disease, response to treatment, relapse of the disease, or recurrence of the disease.

As used herein, the term "cancer" refers to bladder cancer (BlCa). In some instances, "cancer" is broadly referred to herein by using the term "disease".

As used herein, the term "cancer disease state" refers to any distinguishable manifestation of bladder cancer, including non-cancer. For example, the term includes, without limitation, information regarding the presence or absence of bladder cancer, the presence or absence of a preclinical phase of bladder cancer, the risk of having or developing bladder cancer, the stage of bladder cancer, and progression of bladder cancer.

Some further terms and expressions used in this disclosure are explained below in the detailed description of the invention.

### DETAILED DESCRIPTION OF THE INVENTION

The present disclosure is, at least partly, based on studies aiming to identify cancer-related glycovariants of antigens with improved sensitivity over other variants of the same antigens as cancer biomarkers. In accordance with this aim, the present disclosure provides means and methods for determining bladder cancer disease state in a subject who is suspected to suffer from or be at risk of suffering from bladder cancer. Said means and methods are provided especially for screening, diagnosing, prognosing or monitoring bladder cancer.

It has now been surprisingly revealed that some fucosylated glycovariants of integrins, namely alpha subunits thereof specified hereinbelow, can serve as biomarkers of cancer with improved ability to distinguish patients with cancer from subjects without cancer over other species of the same antigens. More specifically, the fucosylated glycan structure whose presence in the glycovariant is indicative of cancer comprises a fucose-α(1-n) structure, i.e. FUCα(1-n) for short, wherein n is 2.

It has also been surprisingly revealed that fucosylated glycovariants, especially those comprising a FUCα(1-n) structure, preferably a FUCα(1-2)Gal structure, of some further protein antigens such as epithelial cell adhesion molecule (EpCAM), claudin-4 and tumor-associated calcium signal transducer 2 (Trop2) may be used as biomarkers of bladder cancer in combination with each other and with the fucosylated integrin species of the invention.

As used herein, the term "antigen^{FUCα(1-n)}" refers broadly to an antigen, comprising a FUCα(1-n) glycan structure, wherein n is an integer selected from 2, 3, 4 and 6. Accordingly, the term "integrin^{FUCcα(1-n)}", for example, refers to glycovariants of integrin comprising a FUCα(1-n) glycan structure. Corresponding terminology may be applied also to other antigens as wells to any integrin species or subunits thereof.

Likewise, the term "antigen^{FUCα(1-2)Gal}" refers broadly to an antigen disclosed herein, comprising a FUCα(1-2)Gal glycan structure. Accordingly, the terms "integrin^{FUCα(1-2)Gal}", "claudin-4^{FUCα(1-2)Gal}", "EpCAM^{FUCα(1-2)Gal}" and "Trop2^{FUCα(1-2)Gal}" refer to glycovariants of integrin, claudin-4. EpCAM and Trop2 comprising a FUCα(1-2)Gal glycan structure, respectively. Corresponding terminology may be applied also to other fucosylated glycan structures within the term "FUCα(1-n)", to other antigens as wells as to any integrin species or subunits thereof.

Integrins (ITGs) are glycosylated integral membrane proteins composed of an alpha chain and a beta chain. In mammals, integrins have twenty-four different alpha subunits and nine different beta subunits. Several types of integrins are located on the surface of cells and extracellular vesicles (EVs), while some subunits of integrins can also be found as soluble proteins which can be detected in bodily fluids as urine. Alpha subunits suitable for use in the present methods include integrin subunit alpha 1 (ITGA1), integrin subunit alpha 3 (ITGA3), integrin subunit alpha 5 (ITGA5), integrin subunit alpha 11 (ITGA11) and integrin subunit alpha V (ITGAV), while examples of beta subunits outside the scope of the claimed invention include integrin subunit beta 1 (ITGB1), integrin subunit beta 2 (ITGB2), integrin subunit beta 4 (ITGB4), integrin subunit beta 5 (ITGB5) and integrin subunit beta 8 (ITGB8). Also heterodimeric integrins such as integrin alpha 2 beta 1 (ITGA2B1) and integrin alpha 3 beta 1 (ITGA3B1) are outside the scope of the claimed invention.

Epithelial cell adhesion molecule (EpCAM), also known as human epithelial glycoprotein-2 is a transmembrane glycoprotein mediating Ca²⁺-independent homotypic cell-cell adhesion in epithelia. EpCAM can be used as a diagnostic marker for various cancers

Claudin-4, also known as CLDN4 or claudin 4, is a protein which belongs to the claudin family. Claudin-4 is a structural and functional transmembrane component of tight junctions and plays an important role in epithelial paracellular fluid and electrolyte transport.

Tumor-associated calcium signal transducer 2 (Trop2, Trop-2), also known as epithelial glycoprotein-1 antigen, is a transmembrane glycoprotein that is upregulated in cancer.

Mucin 1 (MUC1), also known as mucin1, cell surface associated or cancer antigen 15-3 (CA15-3) is a large transmembrane glycoprotein with molecular weight ranging from 500 to 1000 kDa with extracellular portion consisting of highly conserved 20 amino acid repeat units (tandem repeats) within which there are five potential glycosylation sites. The MUC1 antigen is secreted from tumor cells and is a well-established marker for e.g. breast cancer.

Conventional immunoassays are based on the determination of antigen levels in a sample such as serum or plasma by two different monoclonal antibodies which recognize different protein epitopes of the antigen. Such conventional immunoassays may herein be referred to as "antigen-antigen assays".

Accordingly, conventional ITGA3 immunoassays, for example, are based on the determination of ITGA3 antigen levels in a sample such as serum or plasma by two different monoclonal antibodies which recognize different protein epitopes of ITGA3. Such conventional immunoassays may herein be referred to as "ITGA3-ITGA3 assays". Additionally, the term "ITGA3" may refer to the ITGA3 species recognizable by the conventional immunoassays.

Similarly, conventional mucin 1 (MUC1) immunoassays are based on the determination of MUC1 antigen levels in a sample such as serum or plasma by two different monoclonal antibodies which recognize different epitopes of MUC1. Such conventional immunoassays may herein be referred to as "MUC1-MUC1 assays". Additionally, the term "MUC1" may refer to the MUC1 species recognizable by the conventional immunoassay. Although not repeated, corresponding terminology may be used herein also for other conventional immunoassays regardless of the antigen in question.

It is shown herein that detection of certain glycovariants of protein antigens instead of using conventional immunoassays improves the sensitivity by which patients with cancer can be distinguished from subjects without cancer.

In the experiments leading to some embodiments of the present invention, a lectin, namely *Ulex europaeus* agglutinin-I (UEA-I), among a panel of different plant or human lectins tested showed remarkably good discrimination (p=0.007) between ITGA3 derived from bladder cancer (BlCa) patient urine samples and ITGA3 derived from benign controls (Figure 3B). Benign controls were urine samples from benign prostatic hyperplasia (BPH) patients. As known in the art, UEA-I has affinity to FUCα(1-2), especially to terminal FUCα(1-2)Gal. By assaying the samples for ITGA3^{FUCα(1-2)l} by using UEA-I as a tracer, subjects with bladder cancer could be distinguished from benign controls better than by using a conventional ITGA3-ITGA3 assay which had a higher p-value of 0.023 but was also below the p<0.05 limit of significance (Figure 3A).

The results obtained with bladder cancer-derived ITGA3 were confirmed in and extended to patient stratification in an experiment including serum samples from BlCa and benign sources (BPH) as well as healthy subjects. Assaying the samples for ITGA3^{FUCα(1-2)} by an UEA-I-based assay showed remarkably good discrimination between ITGA3 derived from cancerous sources and ITGA3 derived from benign (BPH) (p<0.00001) or healthy (p=0.00003) sources (Figure 4A). Moreover, the UEA-I-based ITGA3^{FUCα(1-2)} assay was shown to be able to detect both muscle invasive BlCa and non-muscle invasive BlCa (Figure 4B), the latter of which has previously been especially difficult to detect with conventional tests available on the market.

Clear discrimination between cancerous (BlCa) and benign sources by UEA-I was seen also in experiments with integrin species other than ITGA3, namely with ITGA1, ITGA2, ITGA5, ITGA6, ITGA11, ITGAV, ITGB2, ITGB5, ITGB8, ITGA2B1 and ITGA3B1, and with the cell transmembrane proteins claudin-4, EpCAM and Trop2. For example, by assaying the samples for ITGAV^{FUCα(1-2)} and claudin-4^{FUCα(1-2)} by using UEA-I as a tracer, subjects with bladder cancer could be distinguished from benign controls with a p-value of 0.020 and 0.030, respectively (Figs 3C and 5C, respectively). In addition, UEA-I showed good discrimination between EpCAM derived from BlCa patient urine samples and EpCAM derived from benign controls. By assaying the samples for EpCAM^{FUCα(1-2)} by using UEA-I as a tracer, subjects with bladder cancer could be distinguished from benign controls with a p-value of 0.003 (Figure 5A). Moreover, by assaying the samples for Trop2^{FUCα(1-2)} by using UEA-I as a tracer, subjects with bladder cancer could be distinguished from benign controls with a p-value of 0.02 (Figure 5B).

Moreover, clear discrimination between pooled samples obtained from various cancerous sources and from non-malignant sources was achieved by assaying the samples for integrin^{FUCα(1-2)}, more specifically for integrin^{FUCα(1-2)Gal} by using UEA-I as a tracer, as is summarized in Table 1 showing relative signal to background ratios as compared to those obtained with non-malignant samples.

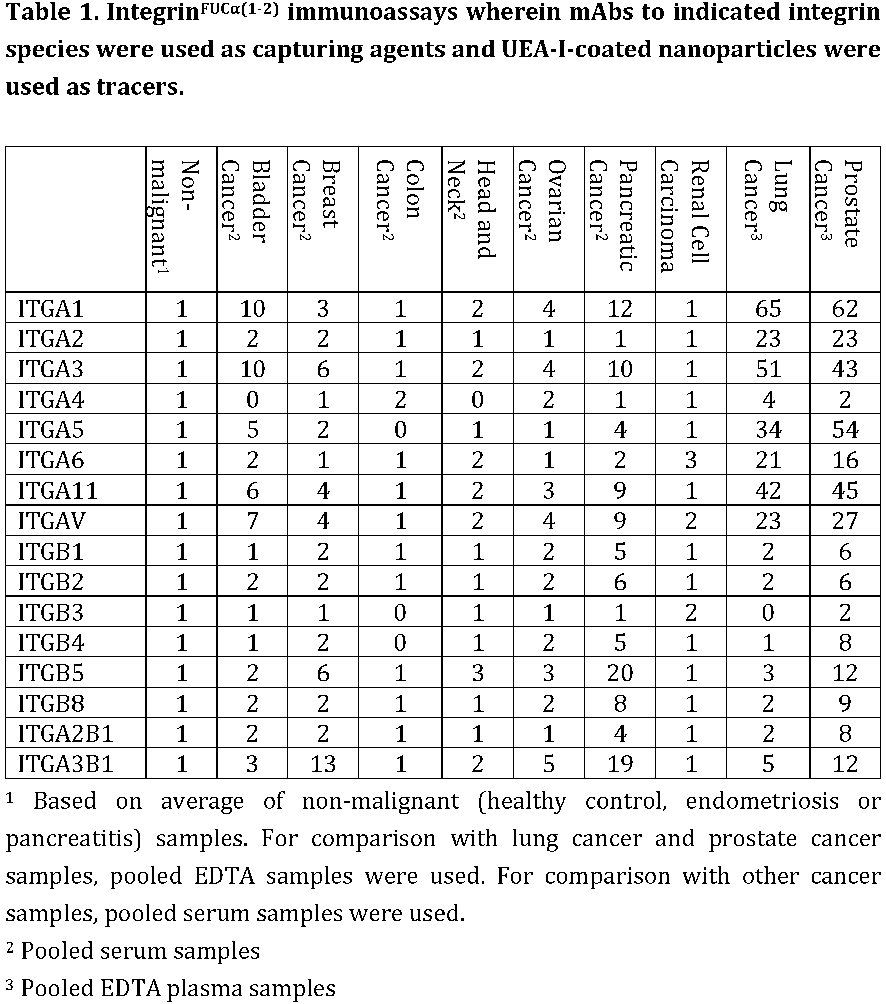

Overall, the present integrin^{FUCα(1-2)} assays performed better than corresponding conventional integrin-integrin immunoassays in distinguishing cancerous samples from non-malignant samples. Some of the results obtained by conventional immunoassays are summarized in Table 2, showing relative signal to background ratios as compared to those obtained with non-malignant samples.

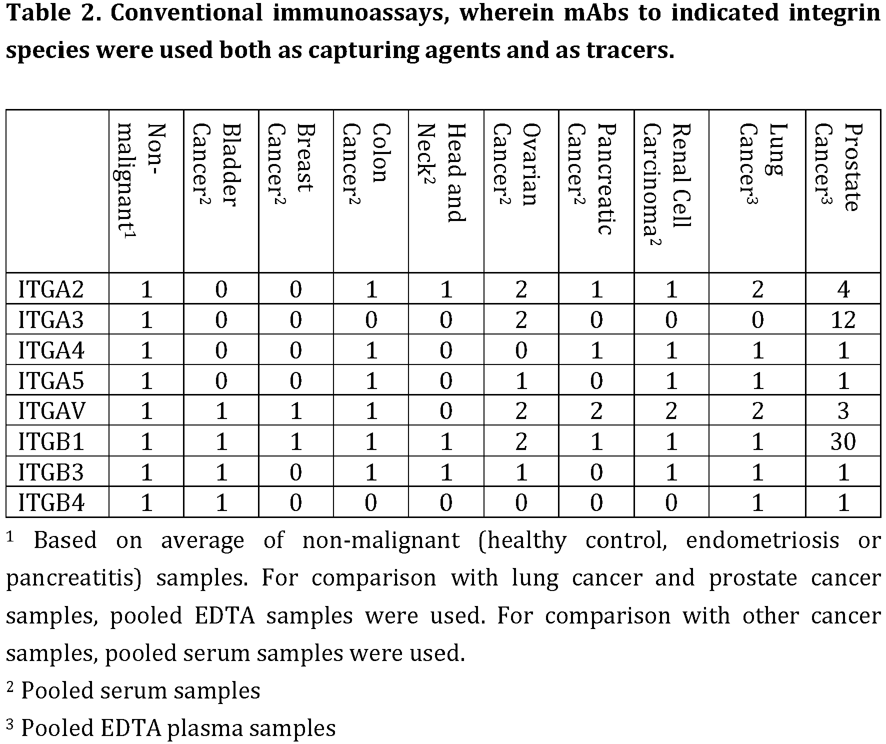

Accordingly, in one aspect, provided herein is use of a FUCα(1-2)Gal glycan structure as a cancer biomarker when comprised on an antigen-presenting extracellular vesicle or on a protein antigen, said antigen being integrin ITGA1, ITGA3, ITGA5, ITGA11, or ITGAV. In some embodiments ITGA3^{FUCα(1-2)GAL} is used either alone or in combination with one or more further integrin^{FUCα(1-2)GAL} species selected independently from ITGA1^{FUCα(1-2)GAL}, ITGA5FUCα(1-2)GAL, ITGA11^{FUCα(1-)GAL}, and ITGAV^{FUCα(1-2)GAL}. In some embodiments ITGA3^{FUCα(1-2)GAL} is used in combination with either ITGA1^{FUCα(1-1)GAL}, ITGA5^{FUCα(1-2)GAL}, ITGA11^{FUCα(1-2)GAL}, or ITGAV^{FUCα(1-2)GAL},

Also provided is a method of determining cancer disease state in a subject, in which method a sample obtained from said subject is first assayed either for the level of integrin-presenting extracellular vesicles comprising a FUCα(1-2)Gal glycan structure or for the level of an integrin antigen glycovariant comprising a FUCα(1-2)Gal structure. In a second step, the assayed level of said vesicles or said antigen glycovariants is compared with that of a control sample or a predetermined threshold value. The subject's cancer diseases state is then determined on the basis of said comparison. In some embodiments increased level of said vesicles or said integrin antigen glycovariants is indicative of cancer. In the method, the integrin antigen is ITGA1, ITGA3, ITGA5, ITGA11, and/or ITGAV. In some embodiments, the integrin antigen is preferably ITGA3.

In some embodiments, the method of determining a cancer disease state in a subject may be more specifically formulated as a method of screening, diagnosing, prognosing and/or monitoring cancer, and/or selecting or assigning treatment for cancer, and/or stratification of cancer patients, be it *de novo* or recurrent appearance or suspicion of cancer.

Moreover, in some embodiments, provided is a method of detecting an integrin-presenting extracellular vesicle comprising a FUCα(1-2)Gal glycan structure or a soluble integrin antigen glycovariant comprising a FUCα(1-2)Gal structure. The method comprises: detecting whether an integrin-presenting extracellular vesicle comprising a FUCα(1-2)Gal glycan structure or an integrin antigen glycovariant comprising a FUCα(1-2)Gal structure is present in a sample obtained from a subject suspected of having cancer or being at risk of having or developing cancer by contacting the sample with an anti-integrin antibody and with a glycan binder specific for FUCα(1-2)Gal, and detecting binding of said glycan binder. In some embodiments, the glycan binder is a lectin capable of binding to a FUCα(1-2)Gal, such as UEA-I, or an anti-FUCα(1-2)Gal antibody. Alternatively, detecting whether the sample obtained from the subject suspected of having cancer or being at risk of having or developing cancer contains said integrin-presenting extracellular vesicle comprising a FUCα(1-2)Gal glycan structure or said integrin antigen glycovariant comprising a FUCα(1-2)Gal structure can be carried out by using mass spectrometry, NMR spectroscopy, electrophoresis or chromatographic methods in any appropriate combination.

Extracellular vesicles (EVs) are lipid bilayer-covered particles that are naturally secreted from cells, and found in biological fluids including, but not limited to, blood, urine and cerebrospinal fluid. EVs carry cargos of proteins, nucleic acids, lipids and metabolites from the parent cell, and may present various antigens such as integrins, mucins (e.g. MUC1), claudin-4, EpCAM and Trop2 on their surfaces. However, these antigens may also be found in biological fluids as soluble proteins, without being comprised on antigen-presenting vesicles. Thus, the present methods encompass determining levels of certain glycan structures in biological samples regardless of whether they exist as glycovariants of soluble antigens or are comprised on antigen-presenting EV's. Thus, assaying a sample for ITGA3^{FUCα1-2GAL}, as an example, refers not only to instances wherein the sample is assayed for the presence of ITGA3 species comprising a FUCα(1-2)Gal glycan structure but also to instances wherein the sample is assayed for EVs presenting both ITGA3 and a FUCα(1-2)Gal glycan structure on its surface. The latter may be achieved by assaying the sample for those EVs that can be captured by an anti-ITGA3 antibody and that comprise a FUCα(1-2)Gal glycan structure on their surface. However, some embodiments may specifically concern soluble glycoforms of antigens, while some other embodiments may specifically concern glycan structures presented by EVs.

The presence of a FUCα(1-2)Gal structure on an integrin antigen or on an integrin-presenting EV may be determined in different ways, especially by utilizing glycan binders such as lectins or anti-glycan antibodies, mass spectrometry, nuclear magnetic resonance (NMR) spectroscopy, electrophoresis or chromatographic methods in any appropriate combination.

Lectins are a group of carbohydrate binding proteins present in many plants, especially seeds, and in fungi, bacteria and animals. For example, *Ulex europaeus* agglutinin-I (UEA-I), also known as *Ulex europaeus* agglutinin-1 (UEA-1) is a 68 kDa fucose-binding plant lectin having affinity to glycan structure Fucα1-2Gal. In the context of this application, the terms UEA, UEA-I and UEA-1 may be used interchangeably.

Accordingly, in some embodiments, determination of the level of FUCα(1-2)Gal structure on an antigen entity, i.e. on an integrin antigen,, or on an integrin-presenting EV, may be carried out with the aid of UEA-I, especially based on detection of UEA-I-binding species of integrins, e.g. by using UEA-I as a tracer. Thus, the term integrin^{FUCα(1-2)GAL} encompasses UEA-I lectin-binding species of said integrin, which may be referred to by the term "integrin^{UEA-I}". However, the term "integrin^{UEA-I}" refers to any glycovariant of said integrin antigen having the capability of binding UEA-I without limitation to the detection technique to be employed or used. In other words, any integrin^{UEA-I} may be detected or assayed not only by techniques employing UEA-I but also by other techniques such as those employing lectins other than UEA-I having the capability of recognizing UEA-I-binding species of said integrin owing to a similar or overlapping binding specificity with that of UEA-I. Specificities of different lectins and, thus, their suitability for this purpose is known to those skilled in the art.

Anti-glycan antibodies specific for the FUCα(1-2)Gal structure may also be employed as binder molecules for detecting any integrin^{Fucα(1-2)Gal} or integrin^{UEA-I} glycoforms. Consequently, the term integrin^{UEA-I} is not limited to the UEA-I-binding species of the integrin antigen detected by an UEA-I-based technique, but encompasses also integrin antigen species having the capability of binding UEA-1 but which are detected by employing another glycan binder, such as an anti-glycan antibody, specific for a glycan structure comprising FUCα(1-2)Gal.

In accordance with what is stated above, the terms "integrin^{UEA-I}" (i.e. ITG^{UEA-I}), "claudin-4^{UEA}", "EpCAM^{UEA-I}" and "Trop2^{UEA-I}" refer to UEA-I lectin binding species of integrin, claudin-4, EpCAM and Trop2, respectively, without limitation to the detection technique. Corresponding terminology applies also to any other antigens, specific integrin species or subunits thereof including, but not limited to integrin alpha subunits ITGA1^{UEA-I}, ITGA2^{UEA-I}, ITGA3^{UEA-I}, ITGA4^{UEA-I}, ITGA5^{UEA-I}, ITGA6^{UEA-I}, ITGA11^{UEA-I} and ITGAV^{UEA-I}, and integrin beta subunits ITGB1^{UEA-I}, ITGB2^{UEA-I}, ITGB6^{UEA-I}, ITGB4^{UEA-I}, ITGB5^{UEA-I} and ITGB8^{UEA-I}, and heterodimeric integrins ITGA2B1^{UEA-I} and ITGA3B1^{UEA-I}. Owing to the interchangeability of the terms antigen^{FUCα(1-2)GAL} and antigen^{UEA-I}, determination of a subject's cancer disease state is in some embodiments based on assaying a sample obtained from said subject for one or more integrin^{FUCα(1-2)GAL} species selected from the group consisting of ITGA1^{FUCα(1-2)GAL}, ITGA3^{FUCα(1-2)GAL}, ITGA5^{FUCα(1-2)GAL}, ITGA11^{FUCα(1-2)GAL}, and ITGAV^{FUCα(1-2)GAL}, with or without assaying the he sample also for claudin-4^{FUCα(1-2)GAL}, EpCAM^{FUCα(1-2)GAL} and Trop2^{FUCα(1-2)GAL}in any combination.

In accordance with the above, the present invention provides a method of determining cancer disease state in a subject by assaying a sample obtained from said subject for integrin^{FUCα(1-2)GAL}. Increased level of said integrin^{FUCα(1-2)GAL} in said sample as compared with that of a control sample or a predetermined threshold value is indicative that said subject has or is at risk of having cancer. On the other hand, non-increased or normal level of integrin^{FUCα(1-2)GAL} in said sample as compared with that of a control sample or a predetermined threshold value is indicative that said subject is apparently healthy with respect to cancer or is not at risk of having or developing cancer.

Likewise, the present invention provides a method of determining cancer disease state in a subject by assaying a sample obtained from said subject for antigen-presenting EVs comprising a FUCα(1-2)Gal glycan structure on their surface, said antigen being selected from integrins namely from ITGA1, ITGA3, ITGA5, ITGA11, and ITGAV. Increased level of said EVs in said sample as compared with that of a control sample or a predetermined threshold value is indicative that said subject has or is at risk of having cancer. On the other hand, non-increased or normal level of said EVs in said sample as compared with that of a control sample or a predetermined threshold value is indicative that said subject is apparently healthy with respect to cancer or is not at risk of having or developing cancer.

The present method of determining a cancer disease state in a subject may also be formulated as a method of identifying a subject as having cancer or being at risk of having or developing cancer, the method comprising a) determining the level of integrin-presenting extracellular vesicles comprising a FUCα(1-2)Gal glycan structure or the level of an integrin antigen glycovariant comprising a FUCα(1-2)Gal structure in a sample obtained from the subject, b) comparing the determined level of said vesicles or said integrin antigen glycovariant of the subject with that of a corresponding level of said vesicles or said integrin antigen glycovariant in a control sample, and c) responsive to the comparison, determining the presence of cancer or the risk of cancer in the subject, wherein increased level of said vesicles or said integrin antigen glycovariant in the sample obtained the subject as compare to the level of said vesicles or said integrin antigen glycovariant in the control indicates that the subject has or is at risk of having or developing cancer. In the method, said integrin is selected from the integrin species specified in claim 1.

It is envisaged that the sensitivity of the present method may be improved by complementing the integrin^{FUCα(1-2)GAL} assay by assaying also for those species of the same antigen that are recognizable by a conventional immunoassay. That means that the sensitivity of the method involving, for example, assaying for ITGA3^{FUCα(1-2)GAL}, may be improved by including ITGA3 as a further biomarker in the method. Accordingly, in some exemplary embodiments concerning ITGA3^{FUCα(1-2)GAL}, the method of determining a subject's cancer disease state may comprise assaying a sample obtained from said subject for ITGA3^{FUCα(1-2)GAL}, and assaying the same or a different sample obtained from said subject for ITGA3 concentration conventionally. Alternatively or in addition, the method may further comprise assaying for any conventional cancer biomarker appropriate for the cancer. For example, without being limited thereto, an appropriate conventional cancer biomarker for BlCa is MUC1. Appropriate conventional biomarkers for any cancer are readily known to those skilled in the art.

In the experiments leading to some embodiments of the present invention, the lectins wheat germ agglutinin (WGA) and *Helix pomatia* agglutinin (HPA) as well as an anti-Tn antigen antibody (Tn-AB) showed significant (p<0.05) discrimination between mucin 1 (MUC1) derived from benign controls and from BlCa patients stratified into low-risk or intermediate & high-risk group. By assaying the samples for MUC1^{WGA}, subjects stratified into the low-risk group could be distinguished from benign controls with a p-value of p=0.01463, and subjects in the intermediate & high-risk group could be distinguished from benign controls with a p=0.02118. By assaying the samples for MUC1^{Tn-AB}, subjects with bladder cancer and stratified into low-risk group or intermediate & high-risk group could be distinguished from benign controls with a p-value of p=0.01831 and p=0.00212, respectively. Discrimination was further improved by constructing a logistic regression model combining data from the MUC1^{WGA} and MUC1^{Tn-AB} assays. Using the model, subjects stratified into the low-risk group could be distinguished from benign controls with a p-value of p=0.00508, and subjects in the intermediate & high-risk group could be distinguished from benign controls with a p=0.00118. A conventional MUC1-MUC1 immunoassay also discriminated subjects in the low-risk or intermediate & high-risk group from benign controls with a p-value of p=0.04006 and p=0.03216, respectively. However, none of the above mentioned MUC1 assays or the model could discriminate between low-risk and intermediate & high-risk groups i.e. p-value was *p*≥0.05. Interestingly, the MUC1^{HPA} assay provided significant discrimination between benign controls and low-risk BlCa group (p=0.03362) and near-significant discrimination (p=0.0548) between low-risk and intermediate & high-risk BlCa group. Thus, MUC1^{HPA} may allow for patient stratification, possibly in combination with other biomarkers including those disclosed herein.

Wheat germ agglutinin (WGA), is a 36 kDa lectin found in wheat. It binds N-acetylglucosamine (GlcNAc) and sialic acid (NeuNAc). Succinylated WGA binds GlcNAc selectively. Accordingly, the term "MUC1^{WGA}" refers to any glycovariant of MUC1 having the capability of binding WGA without limitation to the detection technique to be employed or used. In other words, any MUC1^{WGA} may be detected or assayed not only by techniques employing WGA but also by other techniques such as those employing anti-glycan antibodies having specificity for glycan structures recognizable by WGA, or by mass spectrometry, for instance. Moreover, as specificities of lectins may overlap, MUC1^{WGA} may also be assayed for using other lectins capable of recognizing WGA-binding species of MUC1. Lectins suitable for this purpose are known to those skilled in the art.

*Helix pomatia* agglutinin (HPA) is a 79 kDa N-acetylgalactosamine (GalNAc) binding lectin found in the albumen gland of the roman snail. Accordingly, the term "MUC1^{HPA}" refers to any glycovariant of MUC1 having the capability of binding HPA without limitation to the detection technique to be employed or used. In other words, any MUC1^{HPA} may be detected or assayed not only by techniques employing HPA but also by other techniques such as those employing anti-glycan antibodies having specificity for glycan structures recognizable by HPA, or by mass spectrometry, for instance. Again, as specificities of lectins may overlap, MUC1^{HPA} may also be assayed for using other lectins capable of recognizing HPA-binding species of MUC1. Lectins suitable for this purpose are known to those skilled in the art.

Tn antigen (Tn) refers to the monosaccharide structure N-acetylgalactosamine (GalNAc) linked to serine or threonine by a glycosidic bond. Tn antigen is a neoantigen abnormally expressed in many human cancers. Tn antigen can be targeted by antibodies available from different manufacturers. Accordingly, the term "MUC1^{Tn-AB}" refers to any glycovariant of MUC1 having the capability of binding an anti-Tn antigen antibody without limitation to the detection technique to be employed or used. In other words, any MUC1^{Tn-AB} may be detected or assayed not only by techniques employing an anti-Tn antigen antibody but also by other techniques such as those employing lectins specific for the Tn antigen and mass spectrometry, for instance.

It is envisaged that in some embodiments it would be advantageous to use the integrin^{FUCα(1-2)GAL}, assay, such as the ITGA3^{FUCα(1-2)GAL} assay, in combination with MUC1^{BINDER}, claudin-4^{BINDER}, EpCAM^{BINDER} and/or Trop2^{BINDER} to complement its performance. Preferably, MUC1^{BINDER} is MUC1^{WGA}, MUC1^{Tn-AB} or MUC1^{HPA}, but it may also be any one or more of lectin, anti-glycan antibody or other glycan binder-binding species of MUC1. Preferably, claudin -4^{BINDER} is claudin^{FUCα(1-2)GAL}, but it may also be any one or more of lectin, anti-glycan antibody or other glycan binder-binding species of claudin-4. Preferably, EpCAM^{BINDER} is EpCAM^{FUCα(1-2)GAL}, but it may also be any one or more of lectin, anti-glycan antibody or other glycan binder-binding species of EpCAM. Preferably, Trop2^{BINDER} is Trop2^{FUCα(1-2)GAL}, but it may also be any one or more of lectin, anti-glycan antibody or glycan binder-binding species of Trop2. In other words, said "BINDER" in the general terms MUC1^{BINDER} claudin-4^{BINDER}, EpCAM^{BINDER} and Trop2^{BINDER} may refer to any glycan structure recognizable by one or more lectins preferably selected from the group consisting of UEA-I, AAL, AOL, LCA, SBA, SNA, GSL-1, DC-SIGN, WGA, BPL, DSL, HPA and lectins specific for Tn, or by other glycan binders, such as anti-glycan antibodies, specific for the same glycan structures as said lectins.

It is to be understood that the present methods may involve assaying for one or more integrin^{FUCα(1-2)GAL} species such as ITGA1^{FUCα(1-2)GAL}, ITGA3^{FUCα(1-2)GAL}, ITGA5^{FUCα(1-2)GAL}, ITGA11^{FUCα(1-2)GAÖ}, and ITGAV^{FUCα(1-2)GAL}, in any combination with or without assaying also for one or more integrin protein antigens such as ITGA1, ITGA2, ITGA3, ITGA4, ITGA5, ITGA6, ITGA11, ITGAV, ITGB1, ITGB2, ITGB3, ITGB4, ITGB5, ITGB8, ITGA2B1, ITGA3B1 conventionally. The methods may further comprise assaying for any one or more of EpCAM^{BINDER}, claudin-4^{BINDER}, Trop2^{BINDER} and MUC1^{BINDER} wherein said "BINDER" may refer to any glycan structure recognizable by one or more lectins preferably selected from the group consisting of UEA-I, AAL, AOL, LCA, SBA, SNA, GSL-1, DC-SIGN, WGA, BPL, DSL, HPA and lectins specific for Tn, or other glycan binders, such as anti-glycan antibodies, specific for the same glycan structures as said lectins. Preferably, EpCAM^{BINDER} is EpCAM^{FUCα(1-2)GAL}. Preferably, claudin-4^{BINDER} is claudin^{FUCα(1-2)GAL}. Preferably, Trop2^{BINDER} is Trop2^{FUCα(1-2)GAL}. Preferably, MUC1^{BINDER} is MUC1^{WGA}, MUC1^{Tn-AB}, or MUC1^{HPA}.

In accordance with the above, the present method is in some embodiments directed to diagnosing of cancer, i.e. determining whether or not a subject has or is at risk of having or developing cancer. This is also meant to include instances where the presence or the risk of cancer is not finally determined but that further diagnostic testing is warranted. In such embodiments, the method is not by itself determinative of the presence or absence, or of the risk of cancer in the subject but can indicate that further diagnostic testing is needed or would be beneficial. Therefore, the present method may be combined with one or more other diagnostic methods for the final determination of the presence or absence, or of the risk of cancer in the subject. Such other diagnostic methods are well known to a person skilled in the art.

Being non-invasive and suitable for analysing e.g. urine and serum samples, the present method and its various embodiments may be easily incorporated into a population screening protocol to identify subjects having or being at risk of having or developing cancer. This would enable not only early diagnosis of cancer, but also active surveillance for the onset of cancer in subjects with identified increased risk of developing cancer later in life. Moreover, early detection of cancer would allow treating the disease early when chances of cure are at their highest.

The present method and its various embodiments may be used not only for diagnostic purposes but also for prognosis or predicting the outcome of cancer, or monitoring onset of cancer, any development in risk of cancer, the subject's recovery or survival from cancer, any possible relapse or recurrence of the disease, or response to treatment. In some embodiments, the method comprises monitoring cancer in said subject by comparing the level integrin^{FUCα(1-2)GAL} such as ITGA3^{FUCα(1-2)GAL} as set forth above, with or without concomitantly comparing the level of one or more further biomarkers selected from the group consisting of MUC1^{BINDER}, EpCAM^{BINDER}, claudin-4^{BINDER}, Trop2^{BINDER}, integrin such as ITGA3, and MUC1, with the respective level in one or more other samples obtained from the same subject at a different time point. Samples that may be employed in the monitoring include, but are not limited to, samples collected at different time points after diagnosis of cancer and/or before, during, and after therapeutic intervention, e.g. by surgery, radiation therapy, chemotherapy, any other suitable therapeutic treatment, or any combination thereof, to relieve or cure cancer. In some embodiments, said monitoring is carried out by repeating the assaying step at least twice at different time points, wherein said time points are selected, independently from each other, from the time points set forth above. In some embodiments, the monitoring is carried out during or after treatment of cancer, and/or the method comprises determining said subject as having relapse or recurrence of cancer or as being at risk of relapse or recurrence of cancer, if the level of at least one of the biomarkers is higher than in one or more earlier samples obtained from the same subject, or higher than in a relevant control or above a predetermined threshold value.

In some embodiments, the present method is particularly suitable for early diagnosis of cancer and early detection of cancer relapse, recurrence and progression. Accordingly, integrin^{FUCα(1-2)GAL} such as ITGA3^{FUCα(1-2)GAL}, optionally in combination with one or more biomarkers selected from the group consisting of MUC1^{BINDER}, EpCAM^{BINDER}, Trop2^{BINDER}, integrin such as ITGA3, and MUC1, may serve as early tumor markers for cancer as well as for cancer relapse, recurrence and/or progression. Thus, the present method and any biomarker combinations disclosed herein may be used not only for diagnostic, prognostic and monitoring purposes but also for screening of asymptomatic subjects for cancer or a risk of developing cancer.

For the sake of simplicity, conventional cancer-associated biomarkers disclosed herein, namely integrins, MUC1, claudin-4, EpCAM or Trop2, are collectively referred to by the general term "GlycoProt", while lectin, anti-glycan antibody or other glycan binder-binding glycoforms thereof are referred to by the general term "GlycoProt^{BINDER}". Depending on the embodiment in question, the terms may refer to one or more biomarkers or glycoforms encompassed by the terms, respectively, as is readily understood by those skilled in the art. Preferred GlycoProt^{BINDER} species include, but are not limited to, integrin^{FUCα(1-2)GAL}, MUC1^{WGA} MUC1^{Tn-AB}, MUC1^{HPA}, claudin-4^{FUCα(1-2)GAL}, EpCAM^{FUCα(1-2)GAL} and Trop2^{FUCα(1-2)GAL}

In accordance with the terminology used above, the term "GlycoProt^{BINDER}" encompasses also any GlycoProt-presenting EV that carries a BINDER structure on its surface, but not necessarily on the GlycoProt in question. In accordance with the present invention, such EVs are integrin-presenting vesicles which also carry a FUCα(1-2)Gal structure on their surface.

Assaying a sample for any GlycoProt^{BINDER} disclosed herein may be carried out by any means, methods, or techniques available in the art. A preferred but non-limiting example is to determine the GlycoProt's or the GlycoProt-presenting EV's level of binding to a binder molecule specific for the glycan structure in question. In some embodiments, the binder molecule is lectin or an anti-glycan antibody. This may be performed, for example, by a sandwich assay, wherein a GlycoProt-specific binder molecule, such as an anti-GlycoProt antibody, preferably a monoclonal antibody, is used as a capturing agent and the glycan binder, such as lectin or anti-glycan antibody, in question is used as a tracer. For use as a tracer, said glycan binder may be detectably labelled, either directly or indirectly.

In some other embodiments, a sandwich assay may be conducted using a reversed way. In such cases, the glycan binder, such as lectin or anti-glycan antibody, in question is used as a capturing agent and a GlycoProt-specific binder molecule, such as an antibody, preferably a monoclonal antibody, as a directly or indirectly detectably labelled tracer. Since urine contains less interfering glycosylated molecules than blood, the reversed sandwich assay may operate better with urine samples than with blood samples.

Notably, sandwich assays may be employed regardless of whether the glycan structure whose level is to be assayed is on a soluble antigen or on an antigen-presenting vesicle.

In some embodiments, a sandwich assay may comprise one or more washing steps after a capturing step in order to remove any GlycoProt species or GlycoProt-presenting EVs not specific for the capturing agent. Appropriate washing solutions and conditions (e.g. time and temperature) are known to those skilled in the art.

Sandwich assays according to various embodiments of the present invention may be performed either on a solid surface, such as a microtiter plate, or in lateral flow format. Means and methods for binding a capturing agent to a solid surface, e.g. via a streptavidin-biotin complex, or incorporating a capturing agent to a lateral flow assay are known in the art and readily apparent to a skilled person.

Suitable substrates for use in the present solid phase sandwich assays include, but are not limited to, glass, silica, aluminosilicates, borosilicates, metal oxides such as alumina and nickel oxide, gold, various clays, nitrocellulose or nylon. As indicated above, the substrate may in some embodiments be coated with an appropriate compound to enhance binding of a capturing agent (i.e. either a GlycoProt binder or a glycan binder) to the substrate. In some further embodiments, one or more control binders, such as control antibodies or control lectins, may also be attached to the substrate.

Any one or more, preferably monoclonal, anti-GlycoProt antibodies may be used in the above-mentioned sandwich assays either as capturing agents or as tracers. Non-limiting examples of suitable commercial anti-ITGA3 antibodies include IA3, available at least from R&D Systems (Abingdon, UK), while non-limiting examples of suitable commercial anti-EpCAM antibodies include clone 158206, available at least from R&D Systems (Abingdon, UK). Non-limiting examples of suitable commercial anti-MUC1 antibodies include Ma552 and Ma695, available at least from Fujirebio Diagnostics. Non-limiting examples of suitable commercial anti-ITGAV antibodies include 273210, available at least from R&D Systems (Abingdon, UK). Non-limiting examples of suitable commercial anti-claudin-4 antibodies include 382321, available at least from R&D Systems (Abingdon, UK). Further GlycoProt-specific monoclonal antibodies may be produced according to methods well known in the art. For use as a tracer, an anti-GlycoProt antibody may be labelled with any appropriate label known in the art including, but not limited, to fluorescent labels, bioluminescent labels, chemiluminescent labels. In some embodiments, said anti-GlycoProt antibody may detectably labelled indirectly, for example through immobilization to a detectable nanoparticle.

Anti-glycan antibodies are commercially available from multiple sources. Non-limiting examples of suitable commercial anti-Tn antigen antibodies include SBH-Tn, available at least from SBH Sciences (Natick, MA, USA). Lectins are commercially available from multiple sources. Non-limiting examples include HPA available from ThermoFischer Scientific and WGA available from Vectorlabs (Burlingame, CA).

For use as a tracer, glycan binders, such as lectins or anti-glycan antibodies, may be detectably labelled by various ways as is well known in the art. In some embodiments, one or more glycan binders to be employed for assaying a sample for the level of GlycoProt^{BINDER} may be directly labelled with any available detectable label using standard techniques. In some other embodiments, one or more glycan binders to be employed may be detectably labelled indirectly, for example by immobilizing said one or more glycan binders, such as lectins or anti-glycan antibodies, on a detectable nanoparticle. Such nanoparticle-immobilized binders are called binder-NPs, including e.g. lectin-NPs or anti-glycan antibody-NPs, for short.

As used herein, the term "nanoparticle" (NP) refers to a particle, synthetic or natural, having one or more dimensions, e.g. a diameter, of less than about 1000 nm, e.g. about 500 nm or less, about 100 nm or less, or about 50 nm or less. As used herein, the term "about" refers to a range of values ± 10% of a specified value. For example, the phrase "about 100 nm" includes ± 10% of 100 nm, or from 90 nm to 110 nm. The nanoparticles may generally have a spherical shape but also non-spherical shapes such as ellipsoidal shapes can be used. In some embodiments, all the dimensions of said nanoparticle are less than about 1000 nm, about 500 nm or less, about 100 nm or less, or about 50 nm or less.

A variety of different materials may be utilized in the present nanoparticles. Non-limiting examples of suitable polymers include poly(ethylene glycol) (PEG), polystyrene, polyethylene, poly(acrylic acid), poly(methyl methacrylate) (PMMA), polysaccharides, and copolymers or combinations thereof. Other suitable nanoparticle materials include, but are not limited to, colloidal gold, silver, quantum dots, carbon, porous silicon, and liposomes. Further suitable nanoparticle materials include protein nanoparticles, mineral nanoparticles, glass nanoparticles, nanoparticle crystals, metal nanoparticles, and plastic nanoparticles.

Nanoparticles suitable for use in various embodiments of the present invention or disclosure, may be directly or indirectly qualitatively or quantitatively detectable by any known means. For instance, the nanoparticles may be detectable owing to an inherent quality as in the case of e.g. upconverting nanoparticles (UCNP), resonance particles, quantum dots, and gold particles. In some other embodiments, the nanoparticles can be made detectable e.g. by fluorescent labels, bioluminescent labels, chemiluminescent labels. In some further embodiments, labelling or doping with lanthanides, i.e. luminescent lanthanide ions with luminescence emission in visible or near-infrared or infrared wavelengths and long fluorescence decay, such as europium (III), terbium (III), samarium (III), dysprosium (III), ytterbium (III), erbium (III) and neodynium (III), are preferred means for making the present nanoparticles detectable.

Glycan binders, such as lectins or anti-glycan antibodies, may be immobilized on nanoparticles by any suitable method known in the art, including but not limited to that disclosed in the Examples herein. In those embodiments which involve more than one different glycan binders, including e.g. more than one different lectin and/or more than one different anti-glycan antibody, said different glycan binders may be immobilized either on the same or different nanoparticles in any desired ratios. Notably, said different glycan binders may be of different types (e.g. either lectins, anti-glycan antibodies, antibody mimetics or aptamers) or differ in their glycan specificities, or both.

In some non-limiting embodiments, the most preferred nanoparticles are polystyrene nanoparticles having a diameter of either about 97 nm or about 107 nm. Such nanoparticles are commercially available at least from ThermoFisher Scientific and Seradyn Inc. Further preferred nanoparticles include europium chelate-doped nanoparticles. Advantages of such nanoparticles include i) signal amplification provided by a great number of chelates per particle, ii) strengthened functional affinity (avidity) of the lectins or anti-glycan antibodies to their target glycostructure epitopes enabled by the high density of immobilized lectins or anti-glycan antibodies on the particle, and iii) the glycostructure specificity of the lectins or anti-glycan antibodies used as enabled by creation of the multivalent nanoparticles. However, nanoparticles are only one preferred way of providing adequate avidity effect and signal amplification for carrying out the present invention or disclosure and their various embodiments.

Furthermore, in those embodiments which involve more than one different glycan binders, a sample may be assayed for different GlycoProt^{BINDER} species either in a same assay (i.e. concomitantly) or in different assays (i.e. in parallel), either simultaneously or sequentially. In any such assays, said different glycan binders may have been detectably labelled, either directly or indirectly, with same of different labels. In some embodiments, multiplexing, for example, by using differently labelled nanoparticles bearing different glycan binder species in a single assay may be a preferred format for carrying out any of the methods or embodiments thereof disclosed herein.

In some specific embodiments, one or more different glycan binders immobilized on a same or different nanoparticles labelled with a detectable label such as a lanthanide chelate selected from europium(III), terbium(III), samarium(III), and dysprosium(III) are used as tracers. In some more specific embodiments, europium chelate is used as a detectable label. In a non-limiting preferred embodiment, lectin-NP or anti-glycan antibody-NP is used as a tracer and is doped with about 30000 Eu-chelates. In some other specific embodiments, lectin or anti-glycan antibody in question is attached on upconverting phosphorus (UCP) particles, which are particularly suitable for use as tracers in the lateral flow format.

It is also possible to create a detectable signal by using any available sensor technology. For example, a solid surface may incorporate a recognition element (transducer) capable of converting the binding reaction into a detectable signal with or without the use of label moieties. Different types of transducers can be employed, including those based on electrochemical or optical detection. Detection may also be based on either homogeneous or heterogeneous detection techniques, as is apparent to those skilled in the art.

Coating glycan binders, such as lectins or anti-glycan antibodies, on the surface of nanoparticles instead of coating them onto solid surfaces such as microtiter wells, arrays or sensors, brings about significant benefits in terms of assay performance especially in non-competitive assay formats where the glycan binders are used in combination with specific anti-GlycoProt antibodies. When such specific antibodies are bound to the solid phase, the typically significantly higher affinity of these antibodies as compared to that of glycan binders can be exploited in full to capture the target biomarker molecules onto the solid phase with high efficiency and stability as the first step. Given the high affinity of anti-GlycoProt antibodies, also small target molecules with only one copy of the targeted epitope can be captured with high stability. Once the target molecules are captured, the avidity effect of the glycan binder nanoparticles, i.e. the effect where several adjoining glycan binders can bind to adjoining captured target molecules thereby significantly increasing the binding force compared to singular glycan binder molecules, can also be utilized in full.

Further combined with the fact that the nanoparticles typically allow significant intensification of the measurable signal, glycan binder-NP/solid-phase anti-GlycoProt antibody approach, such as the lectin-NP/solid-phase antibody or anti-glycan antibody-NP/solid-phase antibody approach, optimally combines the high specificity of both binding partners, the high binding force of singular anti-GlycoProt antibodies, the high binding force of multiple adjoining glycan binders on multiple adjoining glycostructures (the avidity effect), and the high level of signal detectable for each bound nanoparticle, resulting in the optimal combination of high sensitivity and high specificity, both in terms of both analytical and clinical attributes.

Furthermore, while glycan binders, such as lectins and anti-glycan antibodies, typically have a high specificity against the target glycostructures (glycoforms), such forms may also exist in other molecules than the targeted one. Therefore, in some preferred embodiments, a wash step is employed between the target molecule antibody capture phase and the glycan binder-NP binding phase, to wash out all non-targeted and unbound molecules that may in some cases comprise the same glycostructures as the targeted molecules and hence pose a risk of unspecific detection based on cross-reactivity between species. Once the specific target molecules are captured by the use of anti-GlycoProt antibodies, and other molecules washed away before the glycan binder binding step, the risk of unspecific binding of unwanted targets by glycan binders, such as lectins or anti-glycan antibodies, becomes eliminated. Such a wash step is also preferred to prevent competition of the target molecule for binding to distant sites around the glycan binder-NP, although this would occur with a low affinity in many cases. However, both the risk of cross-reactivity and distant binding increase when the target molecule has several reactive glycostructures like in the case of many molecules with high molecular weight, such as ITGA3 and other integrin species set forth herein, where adjoining glycan binders can bind to adjoining glycostructures in the same target molecule. In such case the use of the non-competitive glycan binder-NP/solid-phase anti-GlycoProt antibody approach with a wash step before the glycan binder-NP binding reaction results in a significantly more sensitive and target specific assay compared to platforms where the glycan binders are bound to the solid phase, or where the glycan binder-NPs are employed without an intermediate wash step. Similarly, because of an almost complete lack of the avidity effect, assay platforms where the glycan binders (instead of the anti-GlycoProt antibodies) are bound to the solid phase have suboptimal performance with small target molecules.

Moreover, assaying a sample for any glycan structure present in a GlycoProt^{BINDER} or on an GlycoProt-presenting vesicle may be carried out by methods or techniques such as, nuclear magnetic resonance (NMR), electrophoresis, chromatographic methods and mass spectrometry, or any appropriate combinations thereof. Suitable NMR methods include, for example, correlation spectroscopy (COSY), total correlation spectroscopy (TOCSY), nuclear overhauser effect spectroscopy (NOESY) and rotating frame overhauser enhancement spectroscopy (ROESY), all of which methods may be used in either 1D or 2D. Suitable electrophoretic methods include, for example, capillary electrophoresis with laser induced fluorescence (CE-LIF), capillary gel electrophoresis (CGE) and capillary zone electrophoresis (CZE). Non-limiting examples of mass spectrometric methods include fast atom bombardment mass spectrometry (FAB-MS), Fourier transform ion cyclotron resonance mass spectrometry (FTICR-MS), liquid chromatography mass spectrometry (LC-MS), liquid chromatography tandem mass spectrometry (LC-MS/MS), liquid chromatography with electrospray ionization mass spectrometry (LC-ESI-MS), matrix-assisted laser desorption/ionization mass spectrometry (MALDI-MS), matrix-assisted laser desorption/ionization tandem mass spectrometry (MALDI-MS/MS) and matrix-assisted laser desorption/ionization imaging mass spectrometry (MALDI-IMS).

The present disclosure also provides a kit for use in the present methods and various embodiments thereof. In its broadest form, the kit comprises reagents for assaying one or more UEA-I-binding species of cancer-associated glycoprotein biomarkers selected from the group consisting of integrins selected from ITGA1, ITGA3, ITGA5, ITGA11, and ITGAV. In other words, the kit comprises reagents for assaying a sample for any desired i integrin^{UEA-I} species specified above or a combination thereof. For each integrin^{UEA-I} to be assayed, at least one reagent is an integrin-binding agent specific for the integrin in question, such as monoclonal anti-integrin antibody, and at least one of the reagents is a glycan binder, such as UEA-I or an anti-glycan antibody, specific for a FUCα(1-2) Gal glycan structure, preferably immobilized on a nanoparticle. Either said integrin-binding agent or glycan binder is detectably labelled. The glycan binder may be indirectly labelled through a detectable nanoparticle on which it is immobilized.

Optionally, the kit may also comprise reagents for assaying one or more GlycoProt antigens as such, preferably selected from the group consisting of integrins, such as ITGA1, ITGA2, ITGA3, ITGA4, ITGA5, ITGA6, ITGA11, ITGAV, ITGB1, ITGB2, ITGB3, ITGB4, ITGB5, ITGB8, ITGA2B1 or ITGA3B1, MUC1, claudin-4, EpCAM and Trop2. In some embodiments the kit comprises a reagent for assaying ITGA3. In some other embodiments, the kit comprises a reagent for assaying MUC1. In some further embodiments, the kit comprises reagents for assaying for both ITGA3 and MUC1. Non-limiting examples of typical reagents for assaying said conventional GlycoProt antigens include two GlycoProt-binding agents for each conventional GlycoProt antigen to be assayed, such as two monoclonal anti-GlycoProt antibodies, which bind to different epitopes in said GlycoProt. For each specific GlycoProt, one of the binding agents may be the same as the GlycoProt-binding agent provided for assaying a respective Glycoprot^{BINDER}. Nevertheless, one of the GlycoProt-binding agents may be immobilized on a solid surface or provided for use as a capturing agent in lateral flow format, while the other GlycoProt-binding agent may comprise a detectable label.

Accordingly, in some embodiments, the kit is provided for determining a subject's cancer disease state, or for screening, diagnosing, prognosing, or monitoring cancer in said subject and/or selecting or assigning treatment for cancer and/or stratification of cancer patients.

In some embodiments, the kit comprises an integrin-binding agent, such as a monoclonal anti-integrin antibody, and UEA-I, . Either said integrin-binding agent or said UEA-I comprises a detectable label or has been immobilized on a solid surface, such as a microtiter plate. In some further embodiments, streptavidin coating of the plates and biotinylation of the antibody are used for said attaching. Alternative ways of achieving the same are readily available for a skilled person. In some embodiments, said monoclonal anti-integrin antibody is selected from anti-ITGA1, anti-ITGA3, anti-ITGA5, anti-ITGA11, and anti-ITGAV antibodies. In some further embodiments, said UEA-I may be replaced with an anti-FUCα(1-2)Gal antibody or another FUCa(1-2)Gal-specific glycan binder.

In some further embodiments, the kit provided for determining a subject's cancer disease state, or for screening, diagnosing, prognosing, or monitoring cancer in said subject and/or selecting or assigning treatment for cancer and/or stratification of cancer patients may further comprise reagents for assaying additional biomarkers selected from the group consisting of integrin species, MUC1, MUC1^{BINDER}, claudin-4^{BINDER}, EpCAM^{BINDER} and Trop2^{BINDER}. Suitable reagents for these assays become apparent from above. Preferably, said integrin species is selected from ITGA1, ITGA2, ITGA3, ITGA4, ITGA5, ITGA6, ITGA11, ITGAV, ITGB1, ITGB2, ITGB3, ITGB4, ITGB5, ITGB8, ITGA2B1 and ITGA3B1. Preferably, EpCAM^{BINDER} is EpCAM^{FUCα(1-2)GAL}. Preferably, claudin-4^{BINDER} is claudin^{FUCα(1-2)GAL}. Preferably, Trop2^{BINDER} is Trop2^{FUCα(1-2)GAL}. Preferably, MUC1^{BINDER} is MUC1^{WGA}, MUC1^{Tn-AB}, or MUC1^{HPA}.

In cases where the kit comprises reagents for assaying one or more additional biomarkers selected from MUC1^{BINDER}, claudin-4^{BINDER}, EpCAM^{BINDER} and Trop2^{BINDER}, the kit comprises a MUC1, claudin-4, EpCAM and/or Trop2 binding agent, such as a monoclonal anti-MUC1, anti-claudin-4, anti-EpCAM or anti Trop2 antibody, and one or more glycan binders, such as lectin(s) or anti-glycan antibodies, optionally immobilized onto a same or a different nanoparticle. Either said antigen-binding agent or said glycan binder comprises a detectable label or has been immobilized on a solid surface, such as a microtiter plate. In some further embodiments, streptavidin coating of the plates and biotinylation of the binding agent are used for said attaching. Alternative ways of achieving the same are readily available for a skilled person.

In cases where the kit comprises reagents for assaying one or more additional biomarkers selected from integrins (e.g. ITGA3) and MUC1, non-limiting examples of typical reagents for assaying integrin or MUC1 include two integrin (e.g. ITGA3) or MUC1 binding agents, such as two monoclonal antibodies, which bind to different epitopes in their target. One of the binding agents may be the same as the integrin or MUC1 binding agent provided for assaying integrin^{BINDER} (e.g. ITGA3^{BINDER}) or MUC1^{BINDER}, One of the two binding agents may be immobilized on a solid surface or provided for use as a capturing agent in lateral flow format, while the other binding agent may comprise a detectable label.

Optionally, the kit may also comprise a control for comparing to a measured value of integrin (e.g. ITGA3) binding to a glycan binder, such as UEA-I. In some embodiments, the control is a threshold value for comparing to the measured value.

In some further embodiments, the kit may also comprise instructions for performing any method of the present disclosure. In some further embodiments, the kit may also comprise a computer readable medium comprising computer-executable instructions for performing any method of the present disclosure.

In addition to the reagents for assaying a sample for the biomarker combinations set forth above, the kit may also comprise reagents for assaying said samples for any other biomarker, especially for one or more biomarkers associated with any other disease than cancer or other cancers. Thus, the kit may be used not only for screening, diagnosing, prognosing, or monitoring and/or selecting or assigning treatment for cancer and/or stratification of cancer patients in one type of cancer but also for screening, diagnosing, prognosing, or monitoring and/or selecting or assigning treatment for cancer and/or stratification of cancer patients, for example, in other cancers, depending on the specificity and sensitivity of the one or more other biomarkers whose concentrations are to be assayed.

Various details and embodiments of the present method apply also to the present kit, as is readily understood by a skilled person. Thus, properties and features of suitable nanoparticles, for instance, are not repeated herein with respect to the kit.

Also provided is the use of UEA-I, optionally in any combination with WGA, HPA and/or Tn-AB, or the use of an anti-glycan antibody specific for the same glycan structure as UEA-I, optionally in combination with one or more glycan binders specific for the same glycan structures as WGA, HPA or Tn-AB, for determining a state of cancer in a subject, or for screening, diagnosing, prognosing, or monitoring cancer in a subject. Any details and specifics disclosed with respect to the present method and its embodiments apply to the various uses of these biomarkers even though the details and specifics are not repeated herein.

It will be obvious to a person skilled in the art that, as technology advances, the inventive concept can be implemented in various ways. The invention and its embodiments are not limited to the examples described below but may vary within the scope of the claims.

### Examples

### Example 1.

The study was conducted after approval by the Ghent University Hospital ethics committee and in accordance to the guidelines and regulations of the Helsinki Declaration. Participants had given written informed consent. Urine samples from patients with bladder cancer (n=13) were collected by catheterization prior to surgical treatment by transurethral resection of the bladder tumor (TURB) or radical cystectomy. Similarly, age matched urine samples from benign prostatic hyperplasia (BPH) patients (n= 9) were collected prior to transurethral resection of the prostate (TURP) or simple prostatectomy and used as benign controls. All patients were in a fasting state. Both BlCa and benign control (BPH) samples were histologically classified and urological co-morbidities possibly influencing immunoassay results were listed (Table 3). All urine samples were centrifuged at 3,000g (20 min) to remove cellular debris, and stored at -80°C.

**Table 3. Patient characteristics. TURP = Transurethral resection of the prostate; BPH = benign prostatic hyperplasia; SP = Simple prostatectomy; SBC = Suprapubic bladder catheter; TURB = transurethral resection of the bladder tumor.**

| **Benign** | | | | | |
|---|---|---|---|---|---|
| **Sample no** | **Age** | **Sex** | **Biobanking event** | **Disease state** | **Urological comorbidity** |
| 01 | 74 | M | TURP + bladder | BPH | Benign fibroepithelial bladder polyp |
| 02 | 85 | M | TURP | BPH | SBC |
| 03 | 58 | M | SP | BPH | SBC |
| 04 | 70 | M | SP | BPH | None |
| 05 | 73 | M | SP | BPH | Urolithiasis (Large bladder calculus) |
| 06 | 85 | M | SP | BPH | None |
| 07 | 74 | M | SP | BPH | Urolithiasis (Small renal calculus) |
| 08 | 71 | M | TURP | BPH | None |
| 09 | 69 | M | SP | BPH | SBC |

| **Bladder cancer** | | | | | |
|---|---|---|---|---|---|
| **Sample no** | **Age** | **Sex** | **Biobanking event** | **Disease state** | **Urological comorbidity** |
| 10 | 73 | M | TURB | pTa High Grade | BPH |
| 11 | 73 | M | Cystectomy | pT3b | None |
| 12 | 80 | M | Cystectomy | pT4a pN2 | None |
| 13 | 76 | F | Cystectomy | ypT1 High Grade ypN0 | None |
| 14 | 66 | M | Cystectomy + Nephrectomy | ccRCC pT2a + UCC pT4a pN2 | None |
| 15 | 78 | M | Cystectomy | pT2b pN0 | None |
| 16 | 80 | M | TURB | pTis High Grade | None |
| 17 | 68 | F | Cystectomy | ypT2a ypN0 + pTis | None |
| 18 | 54 | M | Cystectomy | pT3b pN1 | None |
| 19 | 63 | M | Cystectomy | pT3b pN0 | None |
| 20 | 68 | M | Cystectomy | pT3b pN1 | None |
| 21 | 64 | M | Cystectomy | ypTa High Grade ypN0 | None |
| 22 | 70 | F | Cystectomy | pT3b pN0 | None |

Monoclonal antibodies anti-ITGA3 (IA3), anti-ITGB1 (4B7R), anti-ITGAV (273210), and anti-ITGA6 (MP4F10) were purchased from R&D systems (Abingdon, UK). The antibodies were biotinylated using a previously described protocol (Islam et al. 2019). Lectins tested in this study and their major glycan-binding specificities are listed in Table 4. Lectins were coated on Eu³⁺-NPs by covalent coupling through their amine groups following a previous protocol (Islam et al. 2019). The Eu³⁺-NPs (Seradyn Indianapolis IN, USA) were monodispersed polystyrene beads.

**Table 4. List of lectins used.**

| **Lectin source** | **Abbreviation** | **Carbohydrate specificity** |
|---|---|---|
| *Ulex europaeus* agglutinin-I | UEA-I | FUCα(1-2)Gal |
| *Aleuria aurantia* lectin | AAL | FUCα(1-3), FUCα(1-6) |
| Soybean agglutinin | SBA | Terminal α-or β linked GalNAc |
| *Sambucus nigra* agglutinin | SNA | sialic acid α (2-6) Gal |
| *Griffonia simplicifolia* lectin-I | GSL-1 | N-acetylgalactosamine, galactose |
| Dendritic Cell-Specific Intercellular adhesion molecule-3-Grabbing Non-integrin | DC-SIGN | Nonsialylated Lewis antigens and high mannose-type structures |
| Wheat germ agglutinin | WGA | Terminal *N*-acetylglucosamine or chitobiose |
| *Bauhinia purpurea* lectin | BPL | N-acetylgalactosamine, lactose. |
| *Datura stramonium* lectin | DSL | (β-1,4) linked N-acetylglucosamine oligomers |
| *Aspergillus oryzae* lectin | AOL | FUCα(1-2), FUCα(1-3), FUCα(1-4), FUCα(1-6) |

The schematic representation of time-resolved fluorescence (TRF) immunoassay is displayed in Figure 1, part b. Briefly, 150 ng of biotinylated capture antibody (e.g. anti-ITGA3, anti-ITGB1, anti-ITGAV or anti-ITGA6) was diluted in assay buffer (Kaivogen, Turku, Finland) in a final volume of 30 µL/well and immobilized in 96-wells-streptavidin-coated plate (KaiSA96, Kaivogen, Turku, Finland) for 1 h at RT. Later, the wells were washed 4 times with wash buffer (Kaivogen, Turku, Finland) using DELFIA plate washer (Perkin-Elmer). Then, 50 µL of urine sample along with 100 µL of assay buffer was added to each well. Urine samples were either from individual BPH or BlCa patients or pooled samples of either BPH (n=9) or BlCa (n=13) patients. After 1 h incubation at RT on a plate shaker at 600 rpm, the wells were washed 4 times with the wash buffer. To each well was added 1 x 10⁷ of lectin conjugated Eu³⁺-NPs (lectin-NPs) in a final volume of 30 µL and incubated for 1.5 h at RT on a plate shaker with slow shaking. In the ITGA3-ITGA3 assay, Eu-NP-conjugated anti-ITGA3 antibody was used as tracer. After that, the wells were washed 4 times with wash buffer in DELFIA plate washer. Signal measurement of the wells was performed (λₑₓ: 340 nm; λₑₘ: 615 nm) with Victor^{™} 1420 multilabel counter (Perkin-Elmer) using the program europium from surface. All assays were conducted in triplicate.

Statistical analysis was performed using GraphPad Prism software, lnc (version 6). The signal-to-background ratios were measured to distinguish between cancer and benign groups. Mann-Whitney U test was also used to compare immunoassay results from BlCa patients and BPH patients. A two-tailed P value < 0.05 indicated statistical significance.

In time-resolved-fluorometry sandwich immunoassays using as tracers a panel of nine lectin-NPs (UEA-I, AAL, SBA, SNA, GSL-1, DC-SIGN, WGA, BPL, and DSL) or Eu-NP-conjugated anti-ITGA3 antibody, distinct binding intensities to the anti-ITGA3 antibody captured antigens were observed through signal to background (S/B) ratios (Figure 2). Conventional ITGA3-ITGA3 assay showed a 2-fold higher S/B ratio with pooled BlCa compared to pooled BPH sample, whereas ITGA3^{UEA-I} assay showed a 6-fold higher S/B ratio with pooled BlCa sample compared to pooled BPH sample. Of the other lectin-NPs, AAL, SNA, GSL-1, DC-SIGN, and BPL showed 1.5- to 3-fold higher S/B ratios with pooled BlCa compared to pooled BPH. The lectin-NPs SBA, WGA and DSL showed no discrimination between pooled BlCa and BPH.

The performance of the integrin-based assay was evaluated using individual urine samples from BlCa and BPH patients. The assays were considered statistically significant when the two-tailed *P* value was < 0.05. The conventional ITGA3-ITGA3 assay showed significant discrimination (Figure 3A, *p*=0.023) of BlCa from BPH, whereas the ITGA3^{UEA-I} assay exhibited clearly improved discrimination of BlCa and BPH (Figure 3B, *p*=0.007) as compared to the conventional assay.

The performance of assays combining capture antibodies for other integrin subunits with UEA-I detection from urine samples of BlCa and BPH patients was also evaluated. For example, ITGAV^{UEA-I} showed significant discrimination of BlCa from BPH (*p*=0.020) as shown in Figure 3C.

### Example 2.

In this study, serum samples from 11 apparently healthy volunteers, 9 benign controls from a benign cohort including patients with BPH and/or other benign urological condition(s), and 18 urothelial bladder cancer (BlCa) patients were used. The apparently healthy volunteers were employees from the Biotechnology Unit, Department of Biochemistry. After signed consent, serum samples from benign cohort and BlCa patients were obtained from Turku prostate cancer consortium. The study was approved by the Ethics Committee of the Hospital District of Southwest Finland (ethical committee statement for BlCa samples: TO3/010/13) and conducted in compliance with the Declaration of Helsinki. Of the 18 BlCa samples, 5, 3, 3 and 7 were classified as Ta low grade, Ta high grade, T1 high grade and <T2 high grade, respectively, according to the tumor-node-metastasis (TNM) classification. Tumors classified as Ta and T1 were considered as non-muscle invasive bladder cancer (NMIBC) and tumors classified as <T2 were considered as muscle invasive bladder cancer (MIBC). Immunoassay of the serum samples (ITGA3-UEA-I) was performed as described above in Example 1.

The ITGA3^{UEA-I} assay significantly (p<0.05) discriminated BlCa from apparently healthy volunteers (p<0.00001) and benign controls (p=0.00003). There was no significant discrimination between apparently healthy volunteers and benign controls (*p*=0.32276) (Figure 4A). In addition, the assay was capable of successfully detecting NMIBC (Ta_low grade, Ta_high grade and T1_high grade) samples as well as MIBC (<T2_high grade) samples (Figure 4B). The detection of NMIBC is often challenging and the current tests available in the market offer poor sensitivity for the detection of NMIBC tumors. However, the detection of glycovariant form of ITGA3 using UEA-I lectin is efficient at detecting even NMIBC tumors.

### Example 3.

Capture antibodies against various cell-adhesion proteins (epithelium cell adhesion molecule (EpCAM), tumor-associated calcium signal transducer 2 (Trop2) and claudin-4 were used to capture these proteins from urine of BlCa (n=13) and benign control (BPH, n=9) groups, and the glycans presented by the proteins were probed using UEA-I. Biotinylated capture antibody for EpCAM (158206, R&D Systems), Trop2 (162-46, BD Biosciences), or claudin-4 (382321, R&D Systems) was prepared as in Example 1 herein and diluted in assay buffer (Kaivogen, Turku, Finland) as 150 ng/30 µL/well and immobilized in 96-streptavidin-coated plate (KaiSA96, Kaivogen, Turku, Finland) for 1 h at RT. Subsequently, the plate was washed 4 times using wash buffer (Kaivogen, Turku, Finland) and DELFIA plate washer (Perkin-Elmer). In each well, 50 µL of urine sample was added diluted with 100 µL of assay buffer. After 1 h incubation at RT on a plate shaker at 600 rpm, the wells were washed 4 times with wash buffer. The detection of captured analyte from urine samples was done by adding 1 x 10⁷/well of nanoparticles conjugated with UEA-I lectin (prepared as in Example 1 herein) in a final volume of 30 µL/well and incubating for 1.5 h at RT on a plate shaker with slow shaking. After that, the wells were washed 4 times with wash buffer in DELFIA plate washer. The signal measurement of wells was performed (λₑₓ: 340 nm; λₑₘ: 615 nm) using VictorTM 1420 multilabel counter (Perkin-Elmer). All assays were conducted in triplicate.

EpCAM^{UEA-I} (*p*=0.003), claudin-4^{UEA-I} (p=0.030) and Trop2^{UEA-I} (*p*=0.02) assays showed statistically significant discrimination (i.e. *p*-value was <0.05) of BlCa patients from benign controls (Figures 5A, 5B and 5C, respectively).

### Example 4

The study was approved by the Ethics Committee of the Hospital District of Southwest Finland (ethical committee statement for BlCa samples: T03/010/13) and conducted in compliance with the Declaration of Helsinki. After signed consent, urine samples, collected via catheter or cystoscope at the time of surgery at the Turku University Hospital, from 14 bladder cancer patients were obtained from Turku prostate cancer consortium. Of the BlCa patients, eight patients underwent transurethral resection of bladder tumor (TURB) (in 2013-2015) and six patients had radical cystectomy (in 2014-2017) at Turku University Hospital. The tumors were graded according to the WHO/International Society of Urinary Pathologist (ISUP) 2004 classifications and staged according to 2010 TNM staging. Further, the patients were also classified into various risk groups such as low-, intermediate-, and high-risk groups according to the guidelines of European Association of Urology (EAU). Briefly, the patients with primary, solitary (<3 cm), low-grade/G1, and Ta are classified as low-risk group. On the other hand, patients with T1, or High-grade/G3, or CIS present, or multiple, recurrent, low- or high-risk criteria are categorized as high-risk group. Patients who do not fit in low- or high-risk groups are classified as intermediate risk group.

For control samples, urine samples from 15 men with clinical suspicion of prostate cancer (PSA 2.5-20 ng/ml and/or abnormal digital rectal examination) in a prospective controlled trial investigating the role of pre-biopsy MRI in prostate cancer diagnosis (IMPROD, ClinicalTrials.gov identifier NCT01864135) were included. Written consent was obtained from the participants. The urine samples were collected directly into sampling containers between 2013 and 2015 at Turku University Hospital. During the median follow up of 0.65 years (24 days to 1.9 years) the status for all of the 15 patients remained unchanged.

All urine samples were stored at -70 °C. The clinicopathological characteristics, including information regarding the patient and tumor characteristics are presented in Table 5.

**Table 5. Clinicopathological characteristics of bladder cancer patients**

| **Characteristic** | | | **Number** |
|---|---|---|---|
| Cases (Bladder cancer) | | | 14 |
| Gender | | Male; Female | 13; 1 |
| Age (Years) | | Median (±SD) | 69.5 (±6.26) |

| **Smoking** | | | |
|---|---|---|---|
| | Never | | 3 |
| | Current | | 6 |
| | Former | | 4 |

| **Survival status** | | | |
|---|---|---|---|
| | Alive, no evidence of disease | | 8 |
| | Alive with cancer relapse | | 1 |
| | Death due to bladder cancer | | 4 |
| | Lost to follow-up | | 1 |

| **WHO_ISUP 2004 grade** | | | |
|---|---|---|---|
| | Low grade | | 5 |
| | High grade | | 9 |

| **pT category** | | | |
|---|---|---|---|
| | pTa; pT1 | | 6; 2 |
| | pT2 | | 1 |
| | pT3; pT4 | | 4; 1 |

| **Risk (According to EAU definition)** | | | |
|---|---|---|---|
| | Low | | 5 |
| | Intermediate | | 3 |
| | High | | 6 |

Anti-MUC1 antibodies Ma552 and Ma695 were obtained from Fujirebio Diagnostics, Gothenburg, Sweden. Biotinylation of Ma552 antibodies was performed as in Example 1 herein. Biotinylated antibodies were stored with 1 g/L BSA at +4 °C. Eu-labelling of Ma695 antibodies was performed by adding 40-fold molar excess of Eu³⁺-chelate. The reaction mixture was incubated overnight at +4 °C. The purification of unreacted Eu³⁺ chelate was done in the same manner as the purification of unreacted biotin-isothiocyanate for biotinylated antibodies in Example 1 herein.

Anti-Tn antigen antibodies were obtained from Fujirebio Diagnostics, Gothenburg, Sweden. Unconjugated *Helix pomatia* agglutinin (HPA) and wheat germ agglutinin (WGA) was purchased from ThermoFischer Scientific and Vectorlabs (Burlingame, CA), respectively. The Eu³⁺-NPs were Fluoro-Max^{™} fluorescent carboxylate-modified particles, 0.1 µm in diameter (Seradyn Inc., Indianapolis, USA). Sulfo-NHS (N-Hydroxysulfosuccinimide sodium salt) was from Sigma-Aldrich and EDC (EDC-HCl Novabiochem^{®}, 1-Ethyl-3-(3'-dimethylaminopropyl)carbodiimide HCl) was from Millipore. Nanosep 300K Omega filters were purchased from Pall Life Sciences and the tip sonicator UP100H and tube sonicator VialTweeter were from Hielscher.

The coating of the Eu³⁺-NPs was performed by utilizing carbodiimide crosslinking chemistry which enables linking the carboxylate groups of the Eu³⁺-NPs to the primary amines of proteins. The steps were performed in room temperature unless mentioned otherwise. All washes were performed with 200-300 µL of buffer by centrifuging the Eu³⁺-NPs in the filters at 5400 × g until no distinct liquid droplet can be observed by eye, but the membrane is still moist. All Eu³⁺-NP recovery steps were performed by sonicating the filter membranes from a 2-5 mm distance for 30 pulses at 60-80% amplitude and recovering the solution from the membrane. Approximately 3.3×10¹¹ Eu³⁺-NPs were used per reaction. The Eu³⁺-NPs were washed twice with 200 µL of conjugation buffer (50 mM MES, pH 6.1) and recovered in 50 µL of conjugation buffer. The particles were activated by vortexing for 15 minutes with EDC and NHS in the final concentration of 1.3 mM and 8 mM, respectively. The Eu³⁺-NPs were conjugated with 150 µg of protein in the reaction buffer (50 mM MES, pH 6.1, 100 mM NaCl) by vortexing for 30 minutes. The pH of the reaction was adjusted to 8.0 with 0.5 M carbonate buffer (pH 9.8) and the mixture was vortexed for 30 minutes. Bovine serum albumin (BSA), for blocking reactive carboxylate groups, was added to a final concentration of 10 mg/mL and the mixture was vortexed for 30 minutes and stored at +4°C overnight in rotary mixing. The mixtures were centrifuged at 5400 × g until dry and washed three times with 300 µL of storage buffer (25 mM Tris, pH 7.8; 150 mM NaCl; 0.1% NaN₃). The particles were recovered in 150 µL of storage buffer, mixed thoroughly, and BSA was added to a final concentration of 2 mg/mL. The Eu³⁺-NPs were stored at +4 °C for two days. The solution was mixed and then sonicated for seven pulses at 100% amplitude. Supernatant was collected after centrifuging at 500 × g for five minutes.

The schematic representation of the time resolved fluorometry (TRF) assays is shown in Fig. 1. In brief, biotinylated antibodies were diluted in Kaivogen assay buffer (Kaivogen, Turku, Finland) to a final concentration of 100 ng/well and were immobilized on streptavidin-coated microtiter wells (Kaivogen) by incubating for 60 minutes at room temperature. The final volume of 50 µL and 25 µL of biotinylated antibody was used when Eu³⁺-chelate labeled Ma695 and anti-Tn antibody or WGA or HPA conjugated Eu³⁺-NPs were used as tracers, respectively. Washing of the wells was performed to remove the excess unbound biotinylated antibody. Then, 50 µL of diluted urine sample was added to the wells in triplicates. Dilution of the urine samples was done in Kaivogen assay buffer. 1:40 dilution of urine sample was performed for the immunoassays where Eu³⁺-chelate labeled Ma695 antibody and Eu³⁺-NPs coated with WGA or HPA were used as tracers. However, the urine samples were diluted 1:20 when Eu³⁺-NPs coated with anti-Tn antibody were used as tracer. The diluted samples were incubated in the wells for 60 minutes on a plate shaker which was followed by washing of the wells. The bound analytes were detected using TRF.

In the conventional MUC1 immunoassay, the sialylated carbohydrate epitope expressed on the MUC1 was targeted by Eu³⁺-chelate labeled MUC1-antibody. The labeled antibodies were diluted in 50 µL of Kaivogen assay buffer and incubated for 60 min at room temperature with shaking. After washing the wells, 200 µL of europium fluorescence intensifier (Kaivogen) was added to each well and incubated on a shaker for 10 min at room temperature.

In assays where the glycan epitopes on MUC1 were targeted using anti-Tn antibody, WGA or HPA conjugated Eu³⁺-NPs, the NP-bioconjugates were diluted in 25 µL of Kaivogen assay buffer for 60 min at room temperature with shaking. The TRF for europium was measured (λₑₓ: 340 nm; λₑₘ: 615 nm) using Victor^{™} 1420 Multilabel counter (Perkin-Elmer Life Sciences).

The specific signals obtained from the conventional MUC1 immunoassay significantly discriminated (i.e. *p*-value was *p*<0.05) between urine samples obtained from patients with benign urological disease, low risk BlCa and intermediate & high risk BlCa (Fig. 6A). However, improved discrimination i.e. lower *p*-values were obtained from detection of cancer-specific glycans on MUC1 such as N-acetylglucosamine (GlcNAc) with WGA lectin or anti-Tn antibody (Tn-AB), and N-acetylgalactosamine (GalNAc) with HPA. The MUC1^{WGA} assay enabled significant discrimination of low risk and intermediate & high risk BlCa from benign urological disease samples (*p*-values: 0.01463 and 0.02118, respectively) (Fig. 6B). However, the low risk BlCa samples could not be differentiated from intermediate & high risk BlCa samples (*p*-value: 0.25143). Similarly, the MUC1^{Tn-AB} assay provided results where the low risk and intermediate & high risk BlCa samples were significantly differentiated from benign samples with *p*-values 0.01831 and 0.00212, respectively, but MUC1^{Tn-AB} assay failed to discriminate low risk samples from intermediate & high risk BlCa samples (*p*-value: 0.25143) (Fig. 6C). A logistic regression model built using the data procured from MUC1^{WGA} and MUC1^{Tn-AB} assays resulted in better discrimination of low risk and intermediate & high risk BlCa from benign samples (*p*-values: 0.00508 and 0.00118, respectively). However, although the model offered a better discrimination of low risk and intermediate & high risk BlCa samples from benign samples, discrimination of low risk BlCa samples from intermediate & high risk BlCa samples was not observed (*p*-value: 0.39358)(Fig. 6D). Contrary to this, the detection of GalNAc on MUC1 using HPA (MUC1^{HPA} assay) yielded significant discrimination of low risk BlCa from benign urological disease (*p*-value: 0.03362) (Fig. 6E). Quite interestingly, the MUC1^{HPA} assay did not offer the discrimination of intermediate & high risk BlCa samples from benign urological disease (*p*-value: 0.0951). Although narrowly above significance limit (*p*-value: 0.0548), the signals from low risk BlCa samples seem to be higher than those of intermediate & high BlCa. Thus, MUC1^{HPA} assay may have potential in distinguishing especially low-risk BlCa, and for example in combination with other biomarkers such as those described herein may provide for BlCa patient stratification into low-risk and intermediate & high-risk groups.

### Example 5

The study was approved by the Ethics Committee of the Hospital District of Southwest Finland, and conducted in compliance with the Declaration of Helsinki. Participants had given written informed consent.

Serum samples obtained from subjects with different cancers (Ovarian, bladder, head and neck, colorectal, pancreatic, Renal cell carcinoma) or benign conditions (endometriosis and pancreatitis) as well as from healthy controls were pooled respectively by combining 6 to 10 individual serum samples. Likewise, 6 to 10 individual EDTA-plasma samples obtained from subjects with cancer (lung and prostate cancer) or from healthy controls were combined to result in respective pooled EDTA plasma samples.

Different anti-human integrin (ITG) monoclonal antibodies (mAb) (anti-ITGA2, anti-ITGA3, anti-ITGA5, anti-ITGAV, anti-ITGB1, anti-ITGB3, anti-ITGB5, and anti-ITGA3B1 mAbs, etc.) recognizing protein epitopes of different integrin subunits were purchased from RnD system (USA). The lectin *Ulex Europaeus* Agglutinin I (UEA I) was obtained from Vector laboratories (USA). Yellow streptavidin (SA) coated low fluorescence microtitration plates, wash buffer, and the assay buffer was from Kaivogen Oy (Turku, Finland). Europium (III)-Chelate-dyed Fluoro-Max^{™} polystyrene nanoparticles (95 nm in diameter, 30,000 chelates per particle, Eu⁺³-NP) were purchased from Seradyn Inc.

Time-resolved fluorometry (TRF) ITG immunoassays (ITG-ITG) and corresponding glycovariant NP assays (ITG-UEA) were carried as follows: biotinylated ITG-mAb captures and Eu-NPs conjugated either with a similar ITG-mAb or α 1,2 Fucose recognizing lectin UEA-I as tracer molecule were used. Each biotinylated capture mAb (80 ng / 25 µl / well) was immobilized on SAv-coated yellow low-fluorescence microtiter wells in a buffer solution by incubating for 1 h at RT. The wells were then washed twice with a wash buffer and 25 µl of diluted pooled serum or EDTA plasma (1:5 in the buffer solution) was added to wells in triplicate and incubated for 1 h at RT with shaking. The immobilized ITGs were detected using two TRF assay formats; the Eu-NP labeled with a similar ITG-mAb detected the ITG protein epitopes whereas the UEA-I lectin-NP conjugates were used for detection of glycan epitopes (α 1,2 Fucose). For the TRF ITG-UEA assays, 25 µl of assay buffer containing 1×10⁷ Eu⁺³ -NPs coated with lectin UEA was added to each well, and incubated for 1 h at RT with shaking. After the incubation, the wells were washed 6 times with the wash buffer. TRF for Eu⁺³ was measured (λex: 340 nm; λem: 615 nm) from dry wells using Victor^{™} 1420 Multilabel Counter (Perkin-Elmer Life Sciences, Wallac, Turku, Finland). The fluorescence signals were compared to background, where each capture antibody was immobilized to wells and assay buffer was used during sample incubation. The signal to background ratio (S/B) was calculated for each capture antibody with corresponding pooled samples.

The results are summarized in Table 1 above showing relative signal to background ratios as compared to those obtained with non-malignant samples.

### References

Islam Md K, Syed P, Lehtinen L, Leivo J, Gidwani K, Wittfooth S, Pettersson K, Lamminmäki U. A Nanoparticle-Based Approach for the Detection of Extracellular Vesicles. Sci Rep. 2019 Jul 11;9(1):10038. doi: 10.1038/s41598-019-46395-2.

## Claims

1. Use of a FUCα(1-2)Gal glycan structure as a biomarker for bladder cancer, wherein said FUCα(1-2)Gal glycan structure is comprised on an integrin-presenting vesicle or an integrin antigen, wherein the integrin is selected from the group consisting of integrin subunit alpha 3 (ITGA3), integrin subunit alpha 1 (ITGA1), integrin subunit alpha 5 (ITGA5), integrin subunit alpha 11 (ITGA11), and integrin subunit alpha V (ITGAV).

2. A method of determining bladder cancer disease state in a subject, the method comprising:
a) assaying a sample of a bodily fluid obtained from said subject for the level of integrin-presenting vesicles comprising a FUCα(1-2)Gal glycan structure, or for the level of integrin antigen comprising a FUCα(1-2)Gal glycan structure, wherein the integrin is selected from the group consisting of integrin subunit alpha 3 (ITGA3), integrin subunit alpha 1 (ITGA1), integrin subunit alpha 5 (ITGA5), integrin subunit alpha 11 (ITGA11), and integrin subunit alpha V (ITGAV),
b) comparing the assayed level obtained in step a) with that of a control sample or a predetermined threshold value, and
c) determining the cancer disease state on the basis of said comparison.

3. The use according to claim 1 or the method according to claim 2, wherein increased level of said integrin antigen or said integrin-presenting vesicle comprising the FUCα(1-2)Gal glycan structure as compared with that of the control sample or predetermined threshold value indicates that said subject has or is at risk of having bladder cancer.

4. The method according to any one of claims 2-3, wherein said assaying is carried out by using a binder molecule specific for a FUCα(1-2)Gal glycan structure or by using mass spectrometry, nuclear magnetic resonance (NMR) spectroscopy, electrophoresis, chromatography or a combination thereof.

5. The method according to claim 4, wherein the binder molecule is *Ulex europaeus* agglutinin-I (UEA-I) or an antibody specific for the FUCα(1-2)Gal glycan structure.

6. The method according to any one of claims 2-5, further comprising assaying said sample for mucin 1 (MUC1) capable of binding to wheat germ agglutinin (WGA) (MUC1^{WGA}), MUC1 capable of binding to anti-Tn antigen antibody (Tn-AB) (MUC1^{Tn-AB}), and/or MUC1 capable of binding to *Helix pomatia* agglutinin (HPA) (MUC1^{HPA}).

7. A kit for use in determining cancer disease state in a subject, comprising:
an integrin-binding agent and a binder molecule specific for a FUCα(1-2)Gal glycan structure, wherein either said integrin-binding agent or said binder molecule comprises a detectable label, wherein the integrin is selected from the group consisting of integrin subunit alpha 3 (ITGA3), integrin subunit alpha 1 (ITGA1), integrin subunit alpha 5 (ITGA5), integrin subunit alpha 11 (ITGA11), and integrin subunit alpha V (ITGAV).

8. Use of a kit for determining bladder cancer disease state in a subject, wherein the kit comprises:
an integrin-binding agent and a binder molecule specific for a FUCα(1-2)Gal glycan structure, wherein either said antigen-binding agent or said binder molecule comprises a detectable label, wherein the integrin is selected from the group consisting of integrin subunit alpha 3 (ITGA3), integrin subunit alpha 1 (ITGA1), integrin subunit alpha 5 (ITGA5), integrin subunit alpha 11 (ITGA11), and integrin subunit alpha V (ITGAV).

9. Use of a binder molecule specific for a FUCα(1-2)Gal glycan structure for determining the presence or absence of a bladder cancer-associated integrin glycoform in a sample of a bodily fluid.

10. The kit according to claim 7 or the use according to claim 8 or claim 9, wherein the binder molecule is UEA-I or an antibody specific for the FUCα(1-2)Gal glycan structure.

## Patentansprüche

1. Verwendung einer FUCα-(1-2)-Gal-Glycan-Struktur als einen Biomarker für Blasenkrebs, wobei die FUCα-(1-2)-Gal-Glycan-Struktur auf einem integrinpräsentierenden Vesikel oder einem Integrin-Antigen enthalten ist, wobei das Integrin aus der Gruppe ausgewählt ist, bestehend aus Integrin-Untereinheit Alpha 3 (ITGA3), Integrin-Untereinheit Alpha 1 (ITGA1), Integrin-Untereinheit Alpha 5 (ITGA5), Integrin-Untereinheit Alpha 11 (ITGA11) und Integrin-Untereinheit Alpha V (ITGAV).

2. Verfahren zum Bestimmen eines Blasenkrebserkrankungszustands bei einem Subjekt, das Verfahren umfassend:
a) Untersuchen einer Probe einer Körperflüssigkeit, die von dem Subjekt erhalten wird, auf den Gehalt an integrinpräsentierenden Vesikeln, umfassend eine FUCα-(1-2)-Gal-Glycan-Struktur, oder auf den Gehalt des Integrin-Antigens, umfassend eine FUCα-(1-2)-Gal-Glycan-Struktur, wobei das Integrin aus der Gruppe ausgewählt ist, bestehend aus Integrin-Untereinheit Alpha 3 (ITGA3), Integrin-Untereinheit Alpha 1 (ITGA1), Integrin-Untereinheit Alpha 5 (ITGA5), Integrin-Untereinheit Alpha 11 (ITGA11) und Integrin-Untereinheit Alpha V (ITGAV),
b) Vergleichen des untersuchten Gehalts, der in Schritt a) erhalten wird, mit dem einer Kontrollprobe oder einem zuvor bestimmten Schwellenwert und
c) Bestimmen des Krebserkrankungszustands auf der Basis des Vergleichs.

3. Verwendung nach Anspruch 1 oder Verfahren nach Anspruch 2, wobei ein erhöhter Gehalt des Integrin-Antigens oder des integrinpräsentierenden Vesikels, umfassend die FUCα-(1-2)-Gal-Glycan-Struktur im Vergleich mit dem der Kontrollprobe oder dem zuvor bestimmten Schwellenwert angibt, dass das Subjekt Blasenkrebs aufweist oder davon bedroht ist.

4. Verfahren nach einem der Ansprüche 2-3, wobei das Untersuchen durch Verwenden eines Bindemolekül, das für eine FUCα-(1-2)-Gal-Glycan-Struktur spezifisch ist, oder durch Verwenden von Massenspektrometrie, Kernspinresonanzspektroskopie (NMR-Spektroskopie), Elektrophorese, Chromatografie oder einer Kombination davon durchgeführt wird.

5. Verfahren nach Anspruch 4, wobei das Bindemolekül *Ulex europaeus* Agglutinin-I (UEA-I) oder ein Antikörper ist, der spezifisch für die FUCα-(1-2)-Gal-Glycan-Struktur ist.

6. Verfahren nach einem der Ansprüche 2 bis 5, ferner umfassend das Untersuchen der Probe auf Mucin 1 (MUC1), das zum Binden an Weizenkeimagglutinin (WGA) in der Lage ist (MUC1^{WGA}), MUC1, das zum Binden an einen Anti-Tn-Antigen-Antikörper [Tn-AB] in der Lage ist (MUC1^{Tn-AB}), und/oder MUC1, das zum Binden an *Helix pomatia* Agglutinin (HPA) in der Lage ist (MUC1^{HPA}).

7. Kit zur Verwendung bei dem Bestimmen des Krebserkrankungszustands bei einem Subjekts, umfassend:
ein integrinbindendes Mittel und ein Bindemolekül, das spezifisch für eine FUCα-(1-2)-Gal-Glycan-Struktur ist, wobei entweder das integrinbindende Mittel oder das Bindemolekül eine nachweisbare Markierung umfasst, wobei das Integrin aus der Gruppe ausgewählt ist, bestehend aus Integrin-Untereinheit Alpha 3 (ITGA3), Integrin-Untereinheit Alpha 1 (ITGA1), Integrin-Untereinheit Alpha 5 (ITGA5), Integrin-Untereinheit Alpha 11 (ITGA11) und Integrin-Untereinheit Alpha V (ITGAV).

8. Verwendung eines Kits zum Bestimmen des Blasenkrebserkrankungszustands bei einem Subjekt, wobei das Kit umfasst:
ein integrinbindendes Mittel und ein Bindemolekül, das spezifisch für eine FUCα-(1-2)-Gal-Glycan-Struktur ist, wobei entweder das antigenbindende Mittel oder das Bindemolekül eine nachweisbare Markierung umfasst, wobei das Integrin aus der Gruppe ausgewählt ist, bestehend aus Integrin-Untereinheit Alpha 3 (ITGA3), Integrin-Untereinheit Alpha 1 (ITGA1), Integrin-Untereinheit Alpha 5 (ITGA5), Integrin-Untereinheit Alpha 11 (ITGA11) und Integrin-Untereinheit Alpha V (ITGAV).

9. Verwendung eines Bindemoleküls, das für eine FUCα-(1-2)-Gal-Glycan-Struktur spezifisch ist, zum Bestimmen des Vorhandenseins oder Fehlens einer Integrin-Glycoform, die mit Blasenkrebs assoziiert ist, in einer Probe einer Körperflüssigkeit.

10. Kit nach Anspruch 7 oder Verwendung nach Anspruch 8 oder 9, wobei das Bindemolekül UEA-1 oder ein Antikörper ist, der für die FUCα-(1-2)-Gal-Glycan-Struktur spezifisch ist.

## Revendications

1. Utilisation d'une structure glycanique FUCα(1-2)Gal comme un biomarqueur du cancer de la vessie, dans laquelle ladite structure glycanique FUCα(1-2)Gal est comprise dans une vésicule de présentation d'intégrine ou un antigène d'intégrine, dans laquelle l'intégrine est choisie dans le groupe constitué de la sous-unité alpha 3 d'intégrine (ITGA3), de la sous-unité alpha 1 d'intégrine (ITGA1), de la sous-unité alpha 5 d'intégrine (ITGA5), de la sous-unité alpha 11 d'intégrine (ITGA11) et de la sous-unité alpha V d'intégrine (ITGAV).

2. Procédé de détermination de la condition pathologique de cancer de la vessie chez un sujet, le procédé comprenant :
a) l'analyse d'un échantillon de fluide corporel obtenu dudit sujet pour déterminer le niveau de vésicules présentant des intégrines comprenant une structure glycanique FUCα(1-2)Gal, ou pour déterminer le niveau d'antigène d'intégrine comprenant une structure glycanique FUCα(1-2)Gal, dans lequel l'intégrine est choisie dans le groupe constitué de la sous-unité alpha 3 d'intégrine (ITGA3), de la sous-unité alpha 1 d'intégrine (ITGA1), de la sous-unité alpha 5 d'intégrine (ITGA5), de la sous-unité alpha 11 d'intégrine (ITGA11) et de la sous-unité alpha V d'intégrine (ITGAV),
b) la comparaison du niveau de dosage obtenu à l'étape a) à celui d'un échantillon de commande ou à une valeur seuil prédéterminée, et
c) le fait de déterminer la condition de maladie cancéreuse sur la base de ladite comparaison.

3. Utilisation selon la revendication 1 ou procédé selon la revendication 2, dans laquelle l'augmentation du niveau dudit antigène d'intégrine ou de la vésicule de présentation d'intégrine comprenant la structure glycanique FUCα(1-2)Gal par rapport à l'échantillon de commande ou à la valeur seuil prédéterminée indique que ledit sujet a ou risque d'avoir un cancer de la vessie.

4. Procédé selon l'une quelconque des revendications 2 à 3, dans lequel ledit dosage est réalisé à l'aide d'une molécule liante spécifique d'une structure glycanique FUCα(1-2)Gal ou à l'aide de la spectrométrie de masse, la spectroscopie de résonance magnétique nucléaire (RMN), l'électrophorèse, la chromatographie ou une combinaison de celles-ci.

5. Procédé selon la revendication 4, dans lequel la molécule liante est l'agglutinine-I d'*Ulex europaeus* (UEA-I) ou un anticorps spécifique de la structure glycanique FUCα(1-2)Gal.

6. Procédé selon l'une quelconque des revendications 2 à 5, comprenant en outre ledit dosage dudit échantillon pour la mucine 1 (MUC1) capable de se lier à l'agglutinine de germe de blé (WGA) (MUC1 ^{WGA}), la MUC1 capable de se lier à l'anticorps antiantigène Tn [Tn-AB] (MUC1^{Tn-AB}), et/ou la MUC1 capable de se lier à l'agglutinine *Helix pomatia* (HPA) (MUC1^{HPA}).

7. Trousse pour utilisation afin de déterminer la condition de maladie cancéreuse chez un sujet, comprenant :
un agent liant l'intégrine et une molécule liante spécifique d'une structure glycanique FUCα(1-2)Gal, dans laquelle ledit agent liant l'intégrine ou ladite molécule liante comprend une étiquette détectable, dans laquelle l'intégrine est choisie dans le groupe constitué de la sous-unité alpha 3 d'intégrine (ITGA3), de la sous-unité alpha 1 d'intégrine (ITGA1), de la sous-unité alpha 5 d'intégrine (ITGA5), de la sous-unité alpha 11 d'intégrine (ITGA11), et de la sous-unité alpha V d'intégrine (ITGAV).

8. Utilisation d'une trousse afin de déterminer la condition de maladie de cancer de la vessie chez un sujet, dans lequel la trousse comprend :
un agent liant l'intégrine et une molécule liante spécifique d'une structure de glycanes FUCα(1-2)Gal, dans laquelle ledit agent liant l'antigène ou ladite molécule liante comprend une étiquette détectable, dans laquelle l'intégrine est choisie dans le groupe constitué de la sous-unité alpha 3 d'intégrine (ITGA3), de la sous-unité alpha 1 d'intégrine (ITGA1), de la sous-unité alpha 5 d'intégrine (ITGA5), de la sous-unité alpha 11 d'intégrine (ITGA11), et de la sous-unité alpha V d'intégrine (ITGAV).

9. Utilisation d'une molécule liante spécifique d'une structure glycannique FUCα(1-2)Gal afin de déterminer la présence ou l'absence d'une glycoforme d'intégrine associée au cancer de la vessie dans un échantillon de fluide corporel.

10. Trousse selon la revendication 7 ou utilisation selon la revendication 8 ou la revendication 9, dans laquelle la molécule liante est UEA-I ou un anticorps spécifique de la structure glycanique FUCα(1-2)Gal.
